# EUROPEAN PATENT APPLICATION

(11) **EP 2 301 971 A1**
(43) Date of publication of application: **30.03.2011**
(21) Application number: 10180146.2
(22) Date of filing: 06.02.2002
(51) Int. Cl.: C07K 16/28, C07K 16/46, A61K 39/395

(54) **Antibodies that bind both BCMA and TACI**

(30) Priority: 20.02.2001 US 270274 P; 12.04.2001 US 283447 P
(62) Divisional of application: 02704362.9
(71) Applicant: ZymoGenetics, L.L.C., Seattle, WA 98102 (US)
(72) Inventor: Kindsvogel, Wayne, R., Seattle, WA 98115 (US)
(74) Representative: Griffin, Philippa Jane

(57) **Abstract**

There is provided an antibody component that specifically binds with both the B-cell maturation antigen (BCMA) and transmembrane activator and calcium-modulator and cyclophilin ligand-interactor (TACI), wherein said antibody component binds to amino acid residues 1-48 of SEQ ID NO:2, and to amino acid residues 30-67 or 68-154 of SEQ ID NO:4.

## Description

### TECHNICAL FIELD

The present invention relates generally to new antibodies that can bind two distinct members of the tumor necrosis factor receptor family. In particular, the present invention provides antibodies that bind both BCMA and TACI proteins, and methods for producing such antibodies.

### BACKGROUND OF THE INVENTION

Cytokines are soluble, small proteins that mediate a variety of biological effects, including the regulation of the growth and differentiation of many cell types (see, for example, Arai et al., Annu. Rev. Biochem. 59:783 (1990); Mosmann, Curr. Opin. Immunol. 3:311 (1991); Paul and Seder, Cell 76:241 (1994)). Proteins that constitute the cytokine group include interleukins, interferons, colony stimulating factors, tumor necrosis factors, and other regulatory molecules. For example, human interleukin-17 is a cytokine that stimulates the expression of interleukin-6, intracellular adhesion molecule 1, interleukin-8, granulocyte macrophage colony-stimulating factor, and prostaglandin E2 expression, and plays a role in the preferential maturation of CD34+ hematopoietic precursors into neutrophils (Yao et al., J. Immunol. 155:5483 (1995); Fossiez et al., J. Exp. Med. 183:2593 (1996)).

Receptors that bind cytokines are typically composed of one or more integral membrane proteins, which bind the cytokine with high affinity and transduce this binding event to the cell through the cytoplasmic portions of the receptor subunits. Cytokine receptors have been grouped into several classes on the basis of similarities in their extracellular ligand binding domains. For example, the receptor chains responsible for binding and/or transducing the effect of interferons are members of the type II cytokine receptor family, based upon a characteristic 200 residue extracellular domain.

Cellular interactions, which occur during an immune response, are regulated by members of several families of cell surface receptors, including the tumor necrosis factor receptor (TNFR) family. The TNFR family consists of a number of integral membrane glycoprotein receptors many of which, in conjunction with their respective ligands, regulate interactions between different hematopoietic cell lineages (see, for example, Cosman, Stem Cells 12:440 (1994); Wajant et al., Cytokine Growth Factor Rev. 10:15 (1999); Yeh et al., Immunol. Rev. 169:283 (1999); Idriss and Naismith, Microsc. Res. Tech. 50:184 (2000)).

One such receptor is "TACI," the transmembrane activator and CAML-interactor (von Bülow and Bram, Science 228:138 (1997); PCT publication WO 98/39361). TACI is a membrane bound receptor, which has an extracellular domain containing two cysteine-rich pseudo-repeats, a transmembrane domain and a cytoplasmic domain that interacts with CAML (calcium-modulator and cyclophilin ligand), an integral membrane protein located at intracellular vesicles, which is a co-inducer of NF-AT activation when overexpressed in Jurkat cells. TACI is associated with B cells and a subset of T cells. Nucleotide sequences that encode TACI and its corresponding amino acid sequence are provided herein as SEQ ID NOs: 3 and 4.

The TACI receptor binds a member of the tumor necrosis factor (TNF) ligand family variously designated as ZTNF4, "BAFF," "neutrokine-α," "BLyS," "TALL-1" and "THANK" (Yu *et al.,* international publication No. WO98/18921 (1998), Moore et al., Science 285:269 (1999); Mukhopadhyay et al., J. Biol. Chem. 274:15978 (1999); Schneider et al., J. Exp. Med. 189:1747 (1999); Shu et al., J. Leukoc. Biol. 65:680 (1999)). The amino acid sequence of ZTNF4 is provided as SEQ ID NO:5. ZTNF4 is also bound by the B-cell maturation antigen (BCMA) (Gross et al., Nature 404:995 (2000)).

Molecules that interfere with the binding between members of the tumor necrosis factor family and their cognate ligands, such as soluble receptors and anti-receptor antibodies, have proven value as therapeutic agents (see, for example, Franklin, Semin. Arthritis Rheum. 29:172 (1999); Bendele et al., Arthritis Rheum. 43:2648 (2000); Kjaergaard et al., Cancer Res. 60:5514 (2000)). The demonstrated in *vivo* activities of antibodies that bind tumor necrosis factor receptors illustrate the clinical potential of, and need for, other such antibodies.

### BRIEF SUMMARY OF THE INVENTION

The present invention provides antibodies that bind two members of the tumor necrosis factor receptor family, and that interrupt ligand binding to these receptors, such as the receptors BCMA, and TACI. More generally, the present invention provides antibodies that are agonists for receptors that bind tumor necrosis factor family ligands (e.g., ZTNF4 and APRIL), or that are antagonists for receptors that bind tumor necrosis factor family ligands. The present invention also provides methods for using such antibodies to inhibit the action of ZTNF4, or tumor necrosis factor family ligands.

### BRIEF DESCRIPTION OF THE DRAWINGS

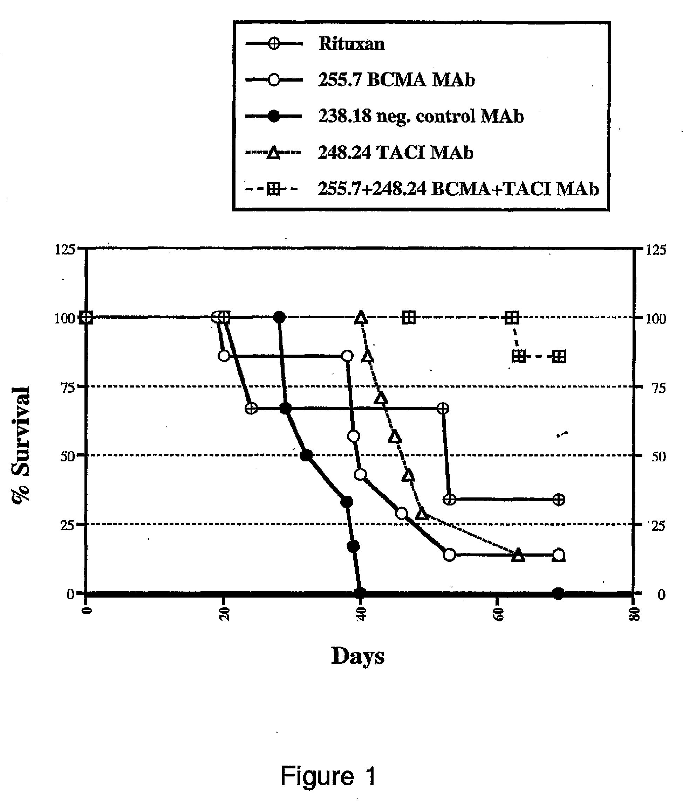

Figure 1 shows survival rates of severe combined immune deficiency (SCID) mice injected with human lymphoma cells. The mice were treated with RITUXAN (a chimeric mouse/human anti-CD20 antibody), a BCMA murine monoclonal antibody ("255.7"), a TACI murine monoclonal antibody ("248.24"), a combination of the BCMA monoclonal antibody 255.7 and TACI monoclonal antibody 248.24, and a negative control murine monoclonal antibody (238.12).

### DETAILED DESCRIPTION OF THE INVENTION

### 1. Overview

Murine monoclonal antibodies were prepared against a polypeptide representing a fragment of the BCMA extracellular domain. Nucleotide sequences that encode BCMA, and its corresponding amino acid sequence are provided herein as SEQ ID NOs: 1 and 2. In an initial study, fluorescence-activated cell sorting analyses with cells that express either BCMA or TACI indicated that a particular monoclonal antibody bound both receptor proteins. This was an unexpected result in view of the low level of amino acid sequence identity shared by the extracellular domains of the two receptors. Enzyme-linked immunosorbent assay analyses of cells expressing either BCMA or TACI confirmed these results. In addition, it was found that preincubation of cells that express either TACI or BCMA with the antibody prevented subsequent binding of ZTNF4 with the receptors. Accordingly, these studies indicated that one antibody could block the binding of ZTNF4 to either BCMA or TACI.

The epitopes bound by the monoclonal antibody were examined using a standard mass spectrometry approach (see, for example, Parker et al., J Immunol. 157:198 (1996)). These studies indicated that the antibody binds a fragment of the extracellular domain of BCMA, represented by amino acid residues 1 to 48 of SEQ ID NO:2, and more particularly, within a region consisting of amino acid residues 13 to 27 of SEQ ID NO:2. The antibody binds two fragments of the TACI extracellular domain, represented by amino acid residues 30 to 67, and 68 to 154 of SEQ ID NO:4. Thus, the antibody appears to recognize a structure within the cysteine-rich regions of both BCMA and TACI proteins. It is therefore possible to generate antibodies that disrupt ligand-receptors interactions of both BCMA and TACI using these extracellular domain fragments as antigens. Such antibodies are useful for targeting mixed populations of cells that express either TACI or BCMA, or both receptors.

As described below, the present invention provides antibody components that specifically bind with both BCMA and TACI receptors. Such anti- BCMA-TACI antibody components bind a polypeptide having the amino acid sequence of amino acid residues 1 to 48 of SEQ ID NO:2 or having the amino acid sequence of amino acid residues 13 to 27 of SEQ ID NO:2, and at least one of: a polypeptide having the amino acid sequence of amino acid residues 30 to 67 of SEQ ID NO:4, and a polypeptide having the amino acid sequence of amino acid residues 68 to 154 of SEQ ID NO:4. Certain anti-BCMA-TACI antibody components can bind: (1) a polypeptide having the amino acid sequence of amino acid residues 1 to 48 of SEQ ID NO:2, or a polypeptide having the amino acid sequence of amino acid residues 13 to 27 of SEQ ID NO:2, (2) a polypeptide having the amino acid sequence of amino acid residues 30 to 67 of SEQ ID NO:4, and (3) a polypeptide having the amino acid sequence of amino acid residues 68 to 154 of SEQ ID NO:4. In addition, certain anti-BCMA-TACI antibody components can bind at least one of: a polypeptide having the amino acid sequence of amino acid residues 39 to 50 of SEQ ID NO:4, and a polypeptide having the amino acid sequence of amino acid residues 78 to 91 of SEQ ID NO:4.

Exemplary antibody components include polyclonal antibodies, murine monoclonal antibodies, humanized antibodies derived from murine monoclonal antibodies, chimeric antibodies, human monoclonal antibodies, and the like. An antibody component can be a whole antibody or an antibody fragment. Illustrative antibody fragments include F(ab')₂, F(ab)₂, Fab', Fab, Fv, scFv, and minimal recognition units.

Particular anti-BCMA-TACI or anti-TACI antibody components can induce a signal via the TACI receptor.

The present invention includes antibody components, such as naked antibodies and naked antibody fragments. In addition, an immunoconjugate can comprise an anti-BCMA-TACI antibody component and a therapeutic agent. Illustrative therapeutic agents include chemotherapeutic drugs, cytotoxins, immunomodulators, chelators, boron compounds, photoactive agents, photoactive dyes, and radioisotopes.

An antibody fusion protein can comprise an anti-BCMA-TACI antibody component and a therapeutic agent, such as an immunomodulator or a cytotoxic polypeptide.

The present invention also contemplates anti-idiotype antibodies, or anti-idiotype antibody fragments, that specifically bind such antibodies or antibody fragments.

The present invention further includes methods for inhibiting in a mammal the activity of ZTNF4 and tumor necrosis factor family ligands, comprising administering to the mammal a composition comprising an antibody component, which specifically binds with the extracellular domain of BCMA and TACI As an illustration, such ZTNF4 activity can be associated with B lymphocytes, activated B lymphocytes, or resting B lymphocytes.

In certain methods, ZTNF4 activity is associated with increased endogenous antibody production. Within related methods, the increased antibody production is associated with an autoimmune disease. Examples of autoimmune diseases include systemic lupus erythematosus, myasthenia gravis, multiple sclerosis, insulin dependent diabetes mellitus, and rheumatoid arthritis. Such diseases can be treated with the anti-receptor antibodies described herein.

In other methods, the ZTNF44 activity is associated with asthma, bronchitis, and emphysema.

In still other methods, the ZTNF4 activity is associated with end stage renal failure or renal disease. Illustrative renal diseases include glomerulonephritis, vasculitis, nephritis, amyloidosis, and pyelonephritis. The activity of ZTNF4 and other tumor necrosis factor family ligands (e.g., APRIL) may alternatively be associated with neoplasms, such as chronic lymphocytic leukemia, multiple myelomas, non-Hodgkin's lymphomas, carcinomas, or light chain gammopathies.

The present invention also includes methods for inhibiting ZTNF4 activity, wherein the ZTNF4 activity is associated with T cells. Within a related method, the ZTNF4 activity is associated with regulating an immune response. Within another method, the ZTNF4 activity is associated with immunosuppression. Within yet another method, the ZTNF4 activity is associated with graft rejection, graft versus host disease, or inflammation. Within still another method, the ZTNF4 activity is associated with autoimmune disease. As an illustration, the autoimmune disease may be insulin-dependent diabetes mellitus or Crohn's disease. In yet other methods, ZTNF4 activity is associated with inflammation. Such inflammation can be associated with, for example, joint pain, swelling, anemia, or septic shock.

The present invention includes methods for inhibiting tumor cell growth with monoclonal antibodies that bind at least one of BCMA and TACI, and that initiate a signal transduced by TACI or BCMA. Certain monoclonal antibodies bind both BCMA and TACI and initiate a signal transduced by TACI or BCMA, while other monoclonal antibodies bind both BCMA and TACI, and initiate a signal transduced by both TACI and BCMA. Antibodies that bind at least one of BCMA or TACI, and that signal may induce apoptosis, activation-induced cell death, cell cycle arrest, or other cytocidal mechanisms.

The present invention also includes methods of inhibiting the proliferation of tumor cells, comprising administering a composition comprising an anti-BCMA-TACI antibody component to the tumor cells. Such treatment can inhibit the growth of a group of tumor cells, *in vitro* or *in vivo,* by killing the tumor cells, or by reducing cellular proliferation. For example, the composition can be administered to cells cultured *in vitro.* As one illustration, the composition can be administered to cells cultured *ex vivo* in conjunction with a bone marrow transplant. In an *in vivo* approach, the composition is a pharmaceutical composition, administered in a therapeutically effective amount to a subject, which has a tumor. Such *in vivo* administration can provide at least one physiological effect selected from the group consisting of decreased number of tumor cells, decreased metastasis, decreased proliferation of tumor cells *(i.e.,* cytostatic), decreased size of a solid tumor, and increased necrosis of a tumor.

The present invention further includes methods of treating a lymphoproliferative disorder in a subject, comprising administering a therapeutically effective amount of pharmaceutical composition to the subject, wherein the pharmaceutical composition comprises an anti-BCMA-TACI antibody component. Illustrative lymphoproliferative disorders include B-cell lymphoma, chronic lymphyocytic leukemia, post-transplantation lymphoproliferative disease, and acute lymphocytic leukemia.

The present invention also provides antibody components, which bind to an antigenic epitope within the stalk region of the TACI receptor. For example, such antibody components can bind a polypeptide consisting of amino acid residues 105 to 166 of SEQ ID NO:4, or a polypeptide consisting of amino acid residues 110 to 118 of SEQ ID NO:4. These antibody components can be administered alone or in conjunction with the administration of anti-BCMA-TACI antibody components.

The present invention further provides methods for inhibiting the proliferation of tumor cells, comprising administering to the tumor cells a multispecific antibody composition, wherein the multispecific antibody composition comprises: (a) an antibody component that binds the extracellular domain of BCMA, and (b) an antibody component that binds the extracellular domain of TACI, wherein the anti-TACI antibody component does not bind the extracellular domain of BCMA, wherein the administration of the multispecific antibody composition inhibits the proliferation of tumor cells. For example, a multispecific antibody composition can be used to inhibit the proliferation of lymphoma cells.

In certain methods, the multispecific antibody composition is administered to cells cultured *in vitro.* In other methods, the multispecific antibody composition is a pharmaceutical composition, and wherein the pharmaceutical composition is administered to a subject, which has a tumor.

A multispecific antibody composition can comprise an anti-BCMA naked antibody component and an anti-TACI naked antibody component, bispecific antibodies that bind BCMA and TACI, and the like. Such compositions can comprise at least one antibody component that further comprises a therapeutic agent. Moreover, a multispecific antibody composition can comprise: (a) an antibody fusion protein that comprises either an immunomodulator or a cytotoxic polypeptide, and (b) at least one of an anti-BCMA antibody component or an anti-TACI antibody component.

These and other aspects of the invention will become evident upon reference to the following detailed description. In addition, various references are identified below.

### 2. Definitions

In the description that follows, a number of terms are used extensively. The following definitions are provided to facilitate understanding of the invention.

As used herein, "nucleic acid" or "nucleic acid molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

The term "complement of a nucleic acid molecule" refers to a nucleic acid molecule having a complementary nucleotide sequence and reverse orientation as compared to a reference nucleotide sequence.

The term "contig" denotes a nucleic acid molecule that has a contiguous stretch of identical or complementary sequence to another nucleic acid molecule. Contiguous sequences are said to "overlap" a given stretch of a nucleic acid molecule either in their entirety or along a partial stretch of the nucleic acid molecule.

The term "degenerate nucleotide sequence" denotes a sequence of nucleotides that includes one or more degenerate codons as compared to a reference nucleic acid molecule that encodes a polypeptide. Degenerate codons contain different triplets of nucleotides, but encode the same amino acid residue (*i.e.,* GAU and GAC triplets each encode Asp).

The term "structural gene" refers to a nucleic acid molecule that is transcribed into messenger RNA (mRNA), which is then translated into a sequence of amino acids characteristic of a specific polypeptide.

An "isolated nucleic acid molecule" is a nucleic acid molecule that is not integrated in the genomic DNA of an organism. For example, a DNA molecule that encodes a growth factor that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

A "nucleic acid molecule construct" is a nucleic acid molecule, either single- or double-stranded, that has been modified through human intervention to contain segments of nucleic acid combined and juxtaposed in an arrangement not existing in nature.

"Linear DNA" denotes non-circular DNA molecules having free 5' and 3' ends. Linear DNA can be prepared from closed circular DNA molecules, such as plasmids, by enzymatic digestion or physical disruption.

"Complementary DNA (cDNA)" is a single-stranded DNA molecule that is formed from an mRNA template by the enzyme reverse transcriptase. Typically, a primer complementary to portions of mRNA is employed for the initiation of reverse transcription. Those skilled in the art also use the term "cDNA" to refer to a double-stranded DNA molecule consisting of such a single-stranded DNA molecule and its complementary DNA strand. The term "cDNA" also refers to a clone of a cDNA molecule synthesized from an RNA template.

A "promoter" is a nucleotide sequence that directs the transcription of a structural gene. Typically, a promoter is located in the 5' non-coding region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (DSEs; McGehee et al., Mol. Endocrinol. 7:551 (1993)), cyclic AMP response elements (CREs), serum response elements (SREs; Treisman, Seminars in Cancer Biol. 1:47 (1990)), glucocorticoid response elements (GREs), and binding sites for other transcription factors, such as CRE/ATF (O'Reilly et al., J. Biol. Chem 267:19938 (1992)), AP2 (Ye et al., J. Biol. Chem. 269:25728 (1994)), SP1, cAMP response element binding protein (CREB; Loeken, Gene Expr. 3:253 (1993)) and octamer factors (see, in general, Watson et al., Eds., Molecular Biology of the Gene, 4th Edition (The Benjamin/Cummings Publishing Company, Inc. 1987), and Lemaigre and Rousseau, Biochem. J. 303:1 (1994)). If a promoter is an inducible promoter, then the rate of transcription increases in response to an inducing agent. In contrast, the rate of transcription is not regulated by an inducing agent if the promoter is a constitutive promoter. Repressible promoters are also known.

A "core promoter" contains essential nucleotide sequences for promoter function, including the TATA box and start of transcription. By this definition, a core promoter may or may not have detectable activity in the absence of specific sequences that may enhance the activity or confer tissue specific activity.

A "regulatory element" is a nucleotide sequence that modulates the activity of a core promoter. For example, a regulatory element may contain a nucleotide sequence that binds with cellular factors enabling transcription exclusively or preferentially in particular cells, tissues, or organelles. These types of regulatory elements are normally associated with genes that are expressed in a "cell-specific," "tissue-specific," or "organelle-specific" manner.

An "enhancer" is a type of regulatory element that can increase the efficiency of transcription, regardless of the distance or orientation of the enhancer relative to the start site of transcription.

"Heterologous DNA" refers to a DNA molecule, or a population of DNA molecules, that does not exist naturally within a given host cell. DNA molecules heterologous to a particular host cell may contain DNA derived from the host cell species (*i.e.,* endogenous DNA) so long as that host DNA is combined with non-host DNA (*i.e.,* exogenous DNA). For example, a DNA molecule containing a non-host DNA segment encoding a polypeptide operably linked to a host DNA segment comprising a transcription promoter is considered to be a heterologous DNA molecule. Conversely, a heterologous DNA molecule can comprise an endogenous gene operably linked with an exogenous promoter. As another illustration, a DNA molecule comprising a gene derived from a wild-type cell is considered to be heterologous DNA if that DNA molecule is introduced into a mutant cell that lacks the wild-type gene.

A "polypeptide" is a polymer of amino acid residues joined by peptide bonds, whether produced naturally or synthetically. Polypeptides of less than about 10 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

A peptide or polypeptide encoded by a non-host DNA molecule is a "heterologous" peptide or polypeptide.

An "integrated genetic element" is a segment of DNA that has been incorporated into a chromosome of a host cell after that element is introduced into the cell through human manipulation. Within the present invention, integrated genetic elements are most commonly derived from linearized plasmids that are introduced into the cells by electroporation or other techniques. Integrated genetic elements are passed from the original host cell to its progeny.

A "cloning vector" is a nucleic acid molecule, such as a plasmid, cosmid, or bacteriophage, which has the capability of replicating autonomously in a host cell: Cloning vectors typically contain one or a small number of restriction endonuclease recognition sites that allow insertion of a nucleic acid molecule in a determinable fashion without loss of an essential biological function of the vector, as well as nucleotide sequences encoding a marker gene that is suitable for use in the identification and selection of cells transformed with the cloning vector. Marker genes typically include genes that provide tetracycline resistance or ampicillin resistance.

An "expression vector" is a nucleic acid molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter.

A "recombinant host" is a cell that contains a heterologous nucleic acid molecule, such as a cloning vector or expression vector. In the present context, an example of a recombinant host is a cell that produces a BCMA or TACI polypeptide from an expression vector. In contrast, a BCMA or TACI polypeptide can be obtained from a cell that is a "natural source" of BCMA or TACI receptors.

"Integrative transformants" are recombinant host cells, in which heterologous DNA has become integrated into the genomic DNA of the cells.

The term "receptor" denotes a cell-associated protein that binds to a bioactive molecule termed a "ligand." This interaction mediates the effect of the ligand on the cell. In the context of receptor binding, the phrase "specifically binds" or "specific binding" refers to the ability of the ligand to competitively bind with the receptor. For example, ZTNF4 specifically binds with the BCMA or TACI receptor, and this can be shown by observing competition for the BCMA or TACI receptor between detectably labeled ZTNF4 and unlabeled ZTNF4.

Receptors can be membrane bound, cytosolic or nuclear; monomeric (e.g., thyroid stimulating hormone receptor, beta-adrenergic receptor) or multimeric (e.g., PDGF receptor, growth hormone receptor, IL-3 receptor, granulocyte macrophage-colony stimulating factor (GM-CSF) receptor, granulocyte-colony stimulating factor (G-CSF) receptor, erythropoietin receptor and IL-6 receptor). Membrane-bound receptors are characterized by a multi-domain structure comprising an extracellular ligand-binding domain and an intracellular effector domain that is typically involved in signal transduction. In certain membrane-bound receptors, the extracellular ligand-binding domain and the intracellular effector domain are located in separate polypeptides that comprise the complete functional receptor.

In general, the binding of ligand to receptor results in a conformational change in the receptor that causes an interaction between the effector domain and other molecule(s) in the cell, which in turn leads to an alteration in the metabolism of the cell. Metabolic events that are often linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids.

The term "secretory signal sequence" denotes a DNA sequence that encodes a peptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

An "isolated polypeptide" is a polypeptide that is essentially free from contaminating cellular components, such as carbohydrate, lipid, or other proteinaceous impurities associated with the polypeptide in nature. Typically, a preparation of isolated polypeptide contains the polypeptide in a highly purified form, *i.e.,* at least about 80% pure, at least about 90% pure, at least about 95% pure, greater than 95% pure, or greater than 99% pure. One way to show that a particular protein preparation contains an isolated polypeptide is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide in alternative physical forms, such as dimers or alternatively glycosylated or derivatized forms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "expression" refers to the biosynthesis of a gene product. For example, in the case of a structural gene, expression involves transcription of the structural gene into mRNA and the translation of mRNA into one or more polypeptides.

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a poly-histidine tract, protein A (Nilsson et al., EMBO J. 4:1075 (1985); Nilsson et al., Methods Enzymol. 198:3 (1991)), glutathione S transferase (Smith and Johnson, Gene 67:31 (1988)), Glu-Glu affinity tag (Grussenmeyer et al., Proc. Natl. Acad. Sci. USA 82:7952 (1985)), substance P, FLAG peptide (Hopp et al., Biotechnology 6:1204 (1988)), streptavidin binding peptide, or other antigenic epitope or binding domain. See, in general, Ford et al., Protein Expression and Purification 2:95 (1991). DNA molecules encoding affinity tags are available from commercial suppliers (e.g., Pharmacia Biotech, Piscataway, NJ).

Antibodies are considered to be "specifically binding" if the antibodies exhibit at least one of the following three properties: (1) antibodies bind to a target protein with a threshold level of binding activity, (2) antibodies do not significantly cross-react with polypeptides related to the target protein, and (3) antibodies bind to cells that express the target protein extracellularly, but the antibodies do not bind to cells that do not express the target protein extracellularly. An example of the third case would be an antibody that binds with the extracellular domain of a receptor. Cell-specific binding of anti-receptor antibodies can be detected by enzyme-linked immunosorbent assay (ELISA), flow cytometry, and the like. In a related approach, an antibody that specifically binds with the ligand binding domain of a receptor can be identified by demonstrating that the binding of the antibody to the receptor can be blocked by pretreating the receptor with ligand.

With regard to the first characteristic, antibodies specifically bind if they bind to a target polypeptide, peptide or epitope with a binding affinity (Kₐ) of 10⁶ M⁻¹ or greater, preferably 10⁷ M⁻¹ or greater, more preferably 10⁸ M⁻¹ or greater, and most preferably 10⁹ M⁻¹ or greater. The binding affinity of an antibody can be readily determined by one of ordinary skill in the art, for example, by Scatchard analysis (Scatchard, Ann. NY Acad. Sci. 51:660 (1949)). With regard to the second characteristic, antibodies do not significantly cross-react with related polypeptide molecules, for example, if they detect BCMA or TACI protein, but not other presently known polypeptides using a standard Western blot analysis. As another example, antibodies that specifically bind with BCMA and TACI receptors do not significantly cross-react with other tumor necrosis factor receptors.

An "immunogenic epitope" is a part of a polypeptide that elicits an antibody response when the polypeptide is used as an immunogen. In contrast, an "antigenic epitope" is a region of a polypeptide to which an antibody can specifically bind. Antigenic epitopes formed by a contiguous sequence of amino acid residues are "linear determinants," whereas "conformational determinants" are formed by amino acid residues that are not in a contiguous sequence, but become spatially juxtaposed in the folded polypeptide.

An "antibody fragment" is a portion of an antibody such as F(ab')₂, F(ab)₂, Fab', Fab, and the like. Regardless of structure, an antibody fragment binds with the same antigen that is recognized by the intact antibody. For example, an antz-BCMA-TACI antibody monoclonal antibody fragment binds with an antigenic epitope of BCMA and TACI.

The term "antibody fragment" also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

A "naked antibody" is an entire antibody, which is not conjugated with a therapeutic agent. Naked antibodies include both polyclonal and monoclonal antibodies, as well as certain recombinant antibodies, such as chimeric and humanized antibodies. Similarly, "naked antibody fragments," are antibody fragments, which are not conjugated with a therapeutic agent.

As used herein, the term "antibody component" includes both an entire antibody and an antibody fragment.

An "anti-BCMA-TACI antibody" is an antibody that specifically binds both BCMA and TACI proteins. Such an antibody can be considered as a "dual reactive" antibody, because it binds two antigens.

A "chimeric antibody" is a recombinant protein that contains the variable domains and complementary determining regions derived from a rodent antibody, while the remainder of the antibody molecule is derived from a human antibody.

"Humanized antibodies" are recombinant proteins in which the complementarity determining regions of a murine monoclonal antibody have been transferred to the heavy and light chain variable regions of a human monoclonal antibody variable domain.

A "bispecific antibody" has binding sites for two different antigens within a single antibody molecule.

A "multispecific antibody composition" comprises antibody components that have binding sites for two different antigens. For example, a multispecific antibody composition can comprise bispecific antibody components, or a multispecific antibody composition can comprise at least two antibody components that bind with different antigens.

As used herein, a "therapeutic agent" is a molecule or atom, which is conjugated to an antibody component to produce a conjugate, which is useful for therapy. Examples of therapeutic agents include drugs, toxins, immunomodulators, chelators, boron compounds, photoactive agents or dyes, and radioisotopes.

A "detectable label" is a molecule or atom, which can be conjugated to an antibody component to produce a molecule useful for diagnosis. Examples of detectable labels include chelators, photoactive agents, radioisotopes, fluorescent agents, paramagnetic ions, or other marker moieties.

An "immunoconjugate" is a conjugate of an antibody component with a therapeutic agent or a detectable label.

As used herein, the term "immunomodulator" includes cytokines, stem cell growth factors, lymphotoxins, co-stimulatory molecules, hematopoietic factors, and the like, as well as synthetic analogs of these molecules.

A "fusion protein" is a hybrid protein expressed by a nucleic acid molecule comprising nucleotide sequences of at least two genes. For example, the term "antibody fusion protein" can refer to a recombinant molecule that comprises an antibody component and a non-antibody therapeutic agent. Examples of therapeutic agents suitable for such fusion proteins include immunomodulators ("antibody-immunomodulator fusion protein") and toxins ("antibody-toxin fusion protein").

An "antigenic peptide" is a peptide, which will bind a major histocompatibility complex molecule to form an MHC-peptide complex, which is recognized by a T cell, thereby inducing a response upon presentation to the T cell. Thus, antigenic peptides are capable of binding to an appropriate major histocompatibility complex molecule and inducing a T cell response, such as cell lysis or specific cytokine release. The antigenic peptide can be bound in the context of a class I or class II major histocompatibility complex molecule, on an antigen presenting cell or on a target cell.

Due to the imprecision of standard analytical methods, molecular weights and lengths of polymers are understood to be approximate values. When such a value is expressed as "about" X or "approximately" X, the stated value of X will be understood to be accurate to ±10%.

### 3. Production of Anti-BCMA-TACI Antibodies

One of skill in the art can produce various anti-BCMA-TACI, "dual reactive," antibodies with the information provided herein. For example, monoclonal or polyclonal antibodies can be produced using at least one polypeptide comprising a cysteine-rich region of either BCMA or TACI, or an immunogenic fragment thereof. Suitable polypeptides derived from BCMA include amino acid residues 1 to 54 of SEQ ID NO:2, amino acid residues 1 to 48 of SEQ ID NO:2, amino acid residues 8 to 41 of SEQ ID NO:2, and amino acid residues 13 to 27 of SEQ ID NO:2. Suitable polypeptides derived from TAGI include the following amino acid residues of SEQ ID NO:4: amino acid residues 30 to 67, amino acid residues 68 to 154, amino acid residues 30 to 154, amino acid residues 34 to 66, and amino acid residues 71 to 104. However, to be considered as an "anti-BCMA-TACI antibody," an antibody produced from such polypeptides must be able to bind both BCMA and TACI. In particular, the antibody must bind BCMA within a region represented by amino acid residues 1 to 54 of SEQ ID NO:2, amino acid residues 1 to 48 of SEQ ID NO:2, or by amino acid residues 13 to 27 of SEQ ID NO:2, and the antibody must bind TACI within a region represented by either amino acid residues 30 to 67 of SEQ ID NO:4, or amino acid residues 68 to 154 of SEQ ID NO:4. Certain anti-BCMA-TACI antibodies bind all three of the following polypeptides: amino acid residues 1 to 48 of SEQ ID NO:2, amino acid residues 30 to 67 of SEQ ID NO:4, and amino acid residues 68 to 154 of SEQ ID NO:4. Other anti-BCMA-TACI antibodies bind all three of the following polypeptides: amino acid residues 13 to 27 of SEQ ID NO:2, amino acid residues 30 to 67 of SEQ ID NO:4, and amino acid residues 68 to 154 of SEQ ID NO:4. In addition, certain anti-BCMA-TACI antibody components can bind at least one of: a polypeptide having the amino acid sequence of amino acid residues 39 to 50 of SEQ ID NO:4, and a polypeptide having the amino acid sequence of amino acid residues 78 to 91 of SEQ ID NO:4.

The resulting antibodies can be screened for the ability to bind both BCMA and TACI using standard techniques, such as an enzyme-linked immunosorbant assay. Standard methods for identifying antigenic epitopes and producing antibodies from amino acid sequences that comprise an immungenic epitope are described, for example, by Mole, "Epitope Mapping," in Methods in Molecular Biology, Vol. 10, Manson (ed.), pages 105-116 (The Humana Press, Inc. 1992), Price, "Production and Characterization of Synthetic Peptide-Derived Antibodies," in Monoclonal Antibodies: Production, Engineering, and Clinical Application, Ritter and Ladyman (eds.), pages 60-84 (Cambridge University Press 1995), Morris (Ed.), Epitope Mapping Protocols (Humana Press, Inc. 1996), and Coligan et al. (eds.), Current Protocols in Immunology, pages 9.3.1 - 9.3.5 and pages 9.4.1- 9.4.11 (John Wiley & Sons 1997).

The antibodies can also be screened for ligand blocking activity using, for example, biotinylated ligands and flow cytometry. Antibodies that block signal transduction by ZTNF4 can be identified by their inhibition of biotin-ZTNF4 binding to BCMA, or TACI, on tumor cell lines. Antibodies that induce a signal by binding to a receptor that binds ZTNF4 can be identified using a suitable reporter cell line that contains a transcriptional reporter element and either TACI or BCMA. Crosslinking of TACI or BCMA by signaling antibodies, for example, can lead to the transcription and translation of the reporter gene product. A fluorescent substrate, such as luciferin, can be used to detect reporter gene expression.

### A. Production of Receptor Polypeptides

TACI or BCMA polypeptides useful for producing antibodies can be produced in recombinant host cells following conventional techniques and receptor sequences disclosed herein. To express a polypeptide-encoding nucleotide sequence, a nucleic acid molecule encoding the polypeptide must be operably linked to regulatory sequences that control transcriptional expression in an expression vector and then, introduced into a host cell. In addition to transcriptional regulatory sequences, such as promoters and enhancers, expression vectors can include translational regulatory sequences and a marker gene, which is suitable for selection of cells that carry the expression vector.

Expression vectors that are suitable for production of a foreign protein in eukaryotic cells typically contain (1) prokaryotic DNA elements coding for a bacterial replication origin and an antibiotic resistance marker to provide for the growth and selection of the expression vector in a bacterial host; (2) eukaryotic DNA elements that control initiation of transcription, such as a promoter; and (3) DNA elements that control the processing of transcripts, such as a transcription termination/polyadenylation sequence. As discussed above, expression vectors can also include nucleotide sequences encoding a secretory sequence that directs the heterologous polypeptide into the secretory pathway of a host cell.

Polypeptides of the present invention may be expressed in mammalian cells. Examples of suitable mammalian host cells include African green monkey kidney cells (Vero; ATCC CRL 1587), human embryonic kidney cells (293-HEK; ATCC CRL 1573), baby hamster kidney cells (BHK-21, BHK-570; ATCC CRL 8544, ATCC CRL 10314), canine kidney cells (MDCK; ATCC CCL 34), Chinese hamster ovary cells (CHO-K1; ATCC CCL61; CHO DG44 (Chasin et al., Som. Cell. Molec. Genet. 12:555, 1986)), rat pituitary cells (GH1; ATCC CCL82), HeLa S3 cells (ATCC CCL2.2), rat hepatoma cells (H-4-II-E; ATCC CRL 1548) SV40-transformed monkey kidney cells (COS-1; ATCC CRL 1650) and murine embryonic cells (NIH-3T3; ATCC CRL 1658).

For a mammalian host, the transcriptional and translational regulatory signals may be derived from viral sources, such as adenovirus, bovine papilloma virus, simian virus, or the like, in which the regulatory signals are associated with a particular gene which has a high level of expression. Suitable transcriptional and translational regulatory sequences also can be obtained from mammalian genes, such as actin, collagen, myosin, and metallothionein genes.

Transcriptional regulatory sequences include a promoter region sufficient to direct the initiation of RNA synthesis. Suitable eukaryotic promoters include the promoter of the mouse *metallothionein I* gene (Hamer et al., J. Molec. Appl. Genet. 1:273 (1982)), the TK promoter of *Herpes* virus (McKnight, Cell 31:355 (1982)), the *SV40* early promoter (Benoist et al., Nature 290:304 (1981)), the *Rous* sarcoma virus promoter (Gorman et al., Proc. Nat'l Acad. Sci. USA 79:6777 (1982)), the cytomegalovirus promoter (Foecking et al., Gene 45:101 (1980)), and the mouse mammary tumor virus promoter (see, generally, Etcheverry, "Expression of Engineered Proteins in Mammalian Cell Culture," in Protein Engineering: Principles and Practice, Cleland et al. (eds.), pages 163-181 (John Wiley & Sons, Inc. 1996)).

Alternatively, a prokaryotic promoter, such as the bacteriophage T3 RNA polymerase promoter, can be used to control gene expression in mammalian cells if the prokaryotic promoter is regulated by a eukaryotic promoter (Zhou et al., Mol. Cell. Biol. 10:4529 (1990), and Kaufman et al., Nucl. Acids Res. 19:4485 (1991)).

An expression vector can be introduced into host cells using a variety of standard techniques including calcium phosphate transfection, liposome-mediated transfection, microprojectile-mediated delivery, electroporation, and the like. The transfected cells can be selected and propagated to provide recombinant host cells that comprise the expression vector stably integrated in the host cell genome. Techniques for introducing vectors into eukaryotic cells and techniques for selecting such stable transformants using a dominant selectable marker are described, for example, by Ausubel (1995) and by Murray (ed.), Gene Transfer and Expression Protocols (Humana Press 1991).

For example, one suitable selectable marker is a gene that provides resistance to the antibiotic neomycin. In this case, selection is carried out in the presence of a neomycin-type drug, such as G-418 or the like. Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A suitable amplifiable selectable marker is dihydrofolate reductase, which confers resistance to methotrexate. Other drug resistance genes (e.g., hygromycin resistance, multi-drug resistance; puromycin acetyltransferase) can also be used. Alternatively, markers that introduce an altered phenotype, such as green fluorescent protein, or cell surface proteins such as CD4, CD8, Class I MHC, placental alkaline phosphatase may be used to sort transfected cells from untransfected cells by such means as FACS sorting or magnetic bead separation technology.

Receptor polypeptides can also be produced by cultured mammalian cells using a viral delivery system. Exemplary viruses for this purpose include adenovirus, herpesvirus, vaccinia virus and adeno-associated virus. Adenovirus, a double-stranded DNA virus, is currently the best studied gene transfer vector for delivery of heterologous nucleic acid (for a review, see Becker et al., Meth. Cell Biol. 43:161 (1994), and Douglas and Curiel, Science & Medicine 4:44 (1997)). Advantages of the adenovirus system include the accommodation of relatively large DNA inserts, the ability to grow to high-titer, the ability to infect a broad range of mammalian cell types, and flexibility that allows use with a large number of available vectors containing different promoters.

Receptor polypeptides can also be expressed in other higher eukaryotic cells, such as avian, fungal, insect, yeast, or plant cells. The baculovirus system provides an efficient means to introduce nucleic acid molecules encoding receptor polypeptides into insect cells. Suitable expression vectors are based upon the *Autographa californica* multiple nuclear polyhedrosis virus, and contain well-known promoters such as *Drosophila heat shock protein* (*hsp*) 70 promoter, *Autographa californica nuclear polyhedrosis virus immediate-early* gene promoter (*ie-1*) and the *delayed early 39K* promoter, baculovirus *p10* promoter, and the *Drosophila metallothionein* promoter. A second method of making recombinant baculovirus utilizes a transposon-based system described by Luckow (Luckow, et al., J. Virol. 67:4566 (1993)). This system, which utilizes transfer vectors, is sold in the BAC-to-BAC kit (Life Technologies, Rockville, MD). This system utilizes a transfer vector, PFASTBAC (Life Technologies) containing a Tn7 transposon to move the DNA encoding a receptor polypeptide into a baculovirus genome maintained in *E. coli* as a large plasmid called a "bacmid." See, Hill-Perkins and Possee, J. Gen. Virol. 71:971 (1990), Bonning, et al., J. Gen. Viral 75:1551 (1994), and Chazenbalk, and Rapoport, J. Biol. Chem. 270:1543 (1995). Using a technique known in the art, a transfer vector containing a receptor polypeptide-encoding sequence is transformed into *E. coli,* and screened for bacmids, which contain an interrupted *lacZ* gene indicative of recombinant baculovirus. The bacmid DNA containing the recombinant baculovirus genome is then isolated using common techniques.

The illustrative PFASTBAC vector can be modified to a considerable degree. For example, the polyhedrin promoter can be removed and substituted with the baculovirus basic protein promoter (also known as P*cor*, p6.9 or MP promoter) which is expressed earlier in the baculovirus infection, and has been shown to be advantageous for expressing secreted proteins (see, for example, Hill-Perkins and Possee, J. Gen. Virol. 71:971 (1990), Bonning, et al., J. Gen. Virol. 75:1551 (1994), and Chazenbalk and Rapoport, J. Biol. Chem. 270:1543 (1995). In such transfer vector constructs, a short or long version of the basic protein promoter can be used.

The recombinant virus or bacmid is used to transfect host cells. Suitable insect host cells include cell lines derived from IPLB-S*f*-21, a *Spodoptera frugiperda* pupal ovarian cell line, such as S*f*9 (ATCC CRL 1711), S*f*21AE, and S*f*21 (Invitrogen Corporation; San Diego, CA), as well as *Drosophila* Schneider-2 cells, and the HIGH FIVEO cell line (Invitrogen) derived from *Trichoplusia ni* (U.S. Patent No. 5,300,435). Commercially available serum-free media can be used to grow and to maintain the cells.

Established techniques for producing recombinant proteins in baculovirus systems are provided by Bailey et al., "Manipulation of Baculovirus Vectors," in Methods in Molecular Biology, Volume 7: Gene Transfer and Expression Protocols, Murray (ed.), pages 147-168 (The Humana Press, Inc. 1991), by Patel et al., "The baculovirus expression system," in DNA Cloning 2: Expression Systems, 2nd Edition, Glover et al. (eds.), pages 205-244 (Oxford University Press 1995), by Ausubel (1995) at pages 16-37 to 16-57, by Richardson (ed.), Baculovirus Expression Protocols (The Human Press, Inc. 1995), and by Lucknow, "Insect Cell Expression Technology," in Protein Engineering: Principles and Practice, Cleland et al. (eds.), pages 183-218 (John Wiley & Sons, Inc. 1996).

Fungal cells, including yeast cells, can also be used to receptor polypeptides described herein. Yeast species of particular interest in this regard include *Saccharomyces cerevisiae, Pichia pastoris,* and *Pichia methanolica.* Suitable promoters for expression in yeast include promoters from *GAL1* (galactose), *PGK* (phosphoglycerate kinase), ADH (alcohol dehydrogenase), *AOX1* (alcohol oxidase), HIS4 (histidinol dehydrogenase), and the like. Many yeast cloning vectors have been designed and are readily available. These vectors include YIp-based vectors, such as YIp5, YRp vectors, such as YRp17, YEp vectors such as YEp13 and YCp vectors, such as YCp19. Methods for transforming *S. cerevisiae* cells with exogenous DNA and producing recombinant polypeptides therefrom are disclosed by, for example, Kawasaki, U.S. Patent No. 4,599,311, Kawasaki et al., U.S. Patent No. 4,931,373, Brake, U.S. Patent No. 4,870,008, Welch et al., U.S. Patent No. 5,037,743, and Murray et al., U.S. Patent No. 4,845,075. Transformed cells are selected by phenotype determined by the selectable marker, commonly drug resistance or the ability to grow in the absence of a particular nutrient (e.g., leucine). A suitable vector system for use in *Saccharomyces cerevisiae* is the *POT1* vector system disclosed by Kawasaki et al. (U.S. Patent No. 4,931,373), which allows transformed cells to be selected by growth in glucose-containing media. Additional suitable promoters and terminators for use in yeast include those from glycolytic enzyme genes (see, e.g., Kawasaki, U.S. Patent No. 4,599,311, Kingsman et al., U.S. Patent No. 4,615,974, and Bitter, U.S. Patent No. 4,977,092) and alcohol dehydrogenase genes. See also U.S. Patents Nos. 4,990,446, 5,063,154, 5,139,936, and 4,661,454.

Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson et al., J. Gen. Microbiol. 132:3459 (1986), and Cregg, U.S. Patent No. 4,882,279. *Aspergillus* cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming *Neurospora* are disclosed by Lambowitz, U.S. Patent No. 4,486,533. The use of *Pichia methanolica* as host for the production of recombinant proteins is disclosed, for example, by Raymond, U.S. Patent No. 5,716,808, Raymond, U.S. Patent No. 5,736,383, Raymond et al., Yeast 14:11 (1998), and in international publication Nos. WO 97/17450, WO 97/17451, WO 98/02536, and WO 98/02565.

Expression vectors can also be introduced into plant protoplasts, intact plant tissues, or isolated plant cells. Methods for introducing expression vectors into plant tissue include the direct infection or co-cultivation of plant tissue with *Agrobacterium tumefaciens,* microprojectile-mediated delivery, DNA injection, electroporation, and the like. See, for example, Horsch et al., Science 227:1229 (1985), Klein et al., Biotechnology 10:268 (1992), and Miki et al., "Procedures for Introducing Foreign DNA into Plants," in Methods in Plant Molecular Biology and Biotechnology, Glick et al. (eds.), pages 67-88 (CRC Press, 1993).

Alternatively, receptor polypeptide-encoding sequences can be expressed in prokaryotic host cells. Suitable promoters that can be used to express the polypeptides in a prokaryotic host are well-known to those of skill in the art and include promoters capable of recognizing the T4, T3, Sp6 and T7 polymerases, the P_{R} and P_{L} promoters of bacteriophage lambda, the *trp, recA,* heat shock, *lacUV5, tac, lpp-lacSpr, phoA,* and *lacZ* promoters of *E. coli,* promoters of *B. subtilis,* the promoters of the bacteriophages of *Bacillus, Streptomyces* promoters, the *int* promoter of bacteriophage lambda, the *bla* promoter of pBR322, and the CAT promoter of the chloramphenicol acetyl transferase gene. Prokaryotic promoters have been reviewed by Glick, J. Ind. Microbiol. 1:277 (1987), Watson et al., Molecular Biology of the Gene, 4th Edition (Benjamin Cummins 1987), and by Ausubel *et al.* (1995).

Suitable prokaryotic hosts include *E. coli* and *Bacillus subtilus.* Suitable strains of *E. coli* include BL21(DE3), BL21(DE3)pLysS, BL21(DE3)pLysE, DH1, DH41, DH5, DH5I, DH5IF, DH5IMCR, DH10B, DH10B/p3, DH11S, C600, HB101, JM101, JM105, JM109, JM110, K38, RR1, Y1088, Y1089, CSH18, ER1451, and ER1647 (see, for example, Brown (ed.), Molecular Biology Labfax (Academic Press 1991)). Suitable strains of *Bacillus subtilus* include BR151, YB886, MI119, MI120, and B170 (see, for example, Hardy, "Bacillus Cloning Methods," in DNA Cloning: A Practical Approach, Glover (ed.) (IRL Press 1985)).

Methods for expressing proteins in prokaryotic hosts are well-known to those of skill in the art (see, for example, Williams et al., "Expression of foreign proteins in E. coli using plasmid vectors and purification of specific polyclonal antibodies," in DNA Cloning 2: Expression Systems, 2nd Edition, Glover et al. (eds.), page 15 (Oxford University Press 1995), Ward et al., "Genetic Manipulation and Expression of Antibodies," in Monoclonal Antibodies: Principles and Applications, page 137 (Wiley-Liss, Inc. 1995), and Georgiou, "Expression of Proteins in Bacteria," in Protein Engineering: Principles and Practice, Cleland et al. (eds.), page 101 (John Wiley & Sons, Inc. 1996)).

Standard methods for introducing expression vectors into bacterial, yeast, insect, and plant cells are provided, for example, by Ausubel (1995).

General methods for expressing and recovering foreign protein produced by a mammalian cell system are provided by, for example, Etcheverry, "Expression of Engineered Proteins in Mammalian Cell Culture," in Protein Engineering: Principles and Practice, Cleland et al. (eds.), pages 163 (Wiley-Liss, Inc. 1996). Standard techniques for recovering protein produced by a bacterial system is provided by, for example, Grisshammer et al., "Purification of over-produced proteins from E. coli cells," in DNA Cloning 2: Expression Systems, 2nd Edition, Glover et al. (eds.), pages 59-92 (Oxford University Press 1995). Established methods for isolating recombinant proteins from a baculovirus system are described by Richardson (ed.), Baculovirus Expression Protocols (The Humana Press, Inc. 1995).

As an alternative, polypeptides of the present invention can be synthesized by exclusive solid phase synthesis, partial solid phase methods, fragment condensation or classical solution synthesis. These synthesis methods are well-known to those of skill in the art (see, for example, Merrifield, J. Am. Chem. Soc. 85:2149 (1963), Stewart et al., "Solid Phase Peptide Synthesis" (2nd Edition), (Pierce Chemical Co. 1984), Bayer and Rapp, Chem. Pept. Prot. 3:3 (1986), Atherton et al., Solid Phase Peptide Synthesis: A Practical Approach (IRL Press 1989), Fields and Colowick, "Solid-Phase Peptide Synthesis," Methods in Enzymology Volume 289 (Academic Press 1997), and Lloyd-Williams et al., Chemical Approaches to the Synthesis of Peptides and Proteins (CRC Press, Inc. 1997)). Variations in total chemical synthesis strategies, such as "native chemical ligation" and "expressed protein ligation" are also standard (see, for example, Dawson et al., Science 266:775 (1994), Hackeng et al., Proc. Nat'l Acad. Sci. USA 94:7845 (1997), Dawson, Methods Enzymol. 287: 34 (1997), Muir et al, Proc. Nat'l Acad. Sci. USA 95:6705 (1998), Severinov and Muir, J. Biol. Chem. 273:16205 (1998), and Kochendoerfer and Kent, Curr. Opin. Chem. Biol. 3:665 (1999)).

### B. Production of Polyclonal Antibodies

Polyclonal antibodies can be prepared using methods well-known to those of skill in the art. See, for example, Green et al., "Production of Polyclonal Antisera," in Immunochemical Protocols (Manson, Ed.), pages 1-5 (Humana Press 1992), and Williams et al., "Expression of foreign proteins in E. coli using plasmid vectors and purification of specific polyclonal antibodies," in DNA Cloning 2: Expression Systems, 2nd Edition, Glover et al. (Eds.), page 15 (Oxford University Press 1995). The immunogenicity of a BCMA or TACI polypeptide can be increased through the use of an adjuvant, such as alum (aluminum hydroxide) or Freund's complete or incomplete adjuvant. Polypeptides useful for immunization also include fusion polypeptides, such as fusions of a BCMA or TACI polypeptide with an immunoglobulin polypeptide or with maltose binding protein. The polypeptide immunogen may be a full-length molecule or a portion thereof. If the polypeptide portion is "hapten-like," such portion may be advantageously joined or linked to a macromolecular carrier (such as keyhole limpet hemocyanin, bovine serum albumin or tetanus toxoid) for immunization.

Although polyclonal antibodies are typically raised in animals such as horses, cows, dogs, chicken, rats, mice, rabbits, guinea pigs, goats, or sheep, an anti-receptor polypeptide antibody of the present invention may also be derived from a subhuman primate antibody. General techniques for raising diagnostically and therapeutically useful antibodies in baboons may be found, for example, in Goldenberg *et al.,* international patent publication No. WO 91/11465, and in Losman et al., Int. J. Cancer 46:310 (1990).

### C. Production of Monoclonal Antibodies

Preferably, monoclonal antibodies to a BCMA or TACI polypeptide are generated. Rodent monoclonal antibodies to specific antigens may be obtained by methods known to those skilled in the art (see, for example, Kohler et al., Nature 256:495 (1975), Harlow and Lane (eds.), Antibodies: A Laboratory Manual (Cold Spring Harbor Laboratory Press 1988); *(*Coligan et al. (eds.), Current Protocols in Immunology, Vol 1, pages 2.5.1-2.6.7 (John Wiley & Sons 1991) ["Coligan"], Picksley et al., "Production of monoclonal antibodies against proteins expressed in E. coli," in DNA Cloning 2: Expression Systems, 2nd Edition, Glover et al. (eds.), page 93 (Oxford University Press 1995), and Goding, Monoclonal Antibodies: Principles and Practice (Academic Press 1996)).

Briefly, monoclonal antibodies can be obtained by injecting mice with a composition comprising a BCMA or TACI polypeptide, verifying the presence of antibody production by removing a serum sample, removing the spleen to obtain Be lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures. Before cloning hybidomas, hybridomas can be screened using a standard technique, such as capture ELISA for BCMA, TACI, or BCMA and TACI.

In a variation of this approach, anti--BCMA-TACI monoclonal antibody can be obtained by fusing myeloma cells with spleen cells from mice immunized with a murine pre-B cell line stably transfected with cDNA encoding one or both of the BCMA and TACI amino acid sequences described herein. This general strategy is described, for example, by Tedder et al., U.S. patent No. 5,484,892 (1996).

An anti-BCMA-TACI antibody can also be derived from a human monoclonal antibody. Human monoclonal antibodies are obtained, for example, from transgenic mice that have been engineered to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described, for example, by Green et al., Nature Genet. 7:13 (1994), Lonberg et al., Nature 368:856 (1994), and Taylor et al., Int. lmmun. 6:579 (1994).

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, Vol. 10, pages 79-104 (The Humana Press, Inc. 1992)).

Anti-BCMA-TACI monoclonal antibodies that initiate a signal via BCMA and TACI may be identified by *in vitro* testing with reporter cell lines. An engineered mammalian cell line (*e.g*., Jurkat), which expresses TACI or BCMA, and a transcriptional reporter gene can be used to test BCMA-TACI monoclonal antibodies for their ability to stimulate transcription of a reporter gene (e.g., luciferase, or a nuclear factor-κB reporter, such as the NF-κB *cis*-reporting system (STRATAGENE; La Jolla, CA)).

### D. Production of Antibody Fragments and Recombinant Antibodies

For particular uses, it may be desirable to prepare fragments of anti-BCMA or anti-TACZ antibodies. Such antibody fragments can be obtained, for example, by proteolytic hydrolysis of the antibody. Antibody fragments can be obtained by pepsin or papain digestion of whole antibodies by conventional methods. As an illustration, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages; As an alternative, an enzymatic cleavage using pepsin produces two monovalent Fab fragments and an Fc fragment directly. These methods are described, for example, by Goldenberg, U.S. patent No. 4,331,647, Nisonoff et al., Arch Biochem. Biophys. 89:230 (1960), Porter, Biochem. J. 73:119 (1959), Edelman et al., in Methods in Enzymology Vol. 1, page 422 (Academic Press 1967), and by Coligan at pages 2.8.1-2.8.10 and 2.10.-2.10.4.

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

For example, Fv fragments comprise an association of V_{H} and V_{L} chains. This association can be noncovalent, as described by Inbar et al., Proc. Nat'l Acad. Sci. USA 69:2659 (1972). Alternatively, the variable chains can be linked by an intermolecular disulfide bond or cross-linked by chemicals such as glutaraldehyde (see, for example, Sandhu, Crit. Rev. Biotech. 12:437 (1992)).

The Fv fragments may comprise V_{H} and V_{L} chains, which are connected by a peptide linker. These single-chain antigen binding proteins (scFv) are prepared by constructing a structural gene comprising DNA sequences encoding the V_{H} and V_{L} domains which are connected by an oligonucleotide. The structural gene is inserted into an expression vector, which is subsequently introduced into a host cell, such as *E. coli.* The recombinant host cells synthesize a single polypeptide chain with a linker peptide bridging the two V domains. Methods for producing scFvs are described, for example, by Whitlow et al., Methods: A Companion to Methods in Enzymology 2:97 (1991) (also see, Bird et al., Science 242:423 (1988), Ladner et al., U.S. Patent No. 4,946,778, Pack et al., Bio/Technology 11:1271 (1993), and Sandhu, Crit. Rev. Biotech. 12:437 (1992)).

As an illustration, an scFV can be obtained by exposing lymphocytes to TACI or BCMA polypeptide *in vitro,* and selecting antibody display libraries in phage or similar vectors (for instance, through use of immobilized or labeled TACI or BCMA protein or peptide). Genes encoding polypeptides having potential BCMA or TACI polypeptide binding domains can be obtained by screening random peptide libraries displayed on phage (phage display) or on bacteria, such as *E. coli.* Nucleotide sequences encoding the polypeptides can be obtained in a number of ways, such as through random mutagenesis and random polynucleotide synthesis. These random peptide display libraries can be used to screen for peptides, which interact with a known target, which can be a protein or polypeptide, such as a ligand or receptor, a biological or synthetic macromolecule, or organic or inorganic substances. Techniques for creating and screening such random peptide display libraries are known in the art (see, for example, Ladner et al., U.S. Patent No. 5,223,409, Ladner et al., U.S. Patent No. 4,946,778, Ladner et al., U.S. Patent No. 5,403,484, Ladner et al., U.S. Patent No. 5,571,698, Kay et al., Phage Display of Peptides and Proteins (Academic Press, Inc. 1996), and Johns, "Phage Display Technology," in Diagnostic and Therapeutic Antibodies, George and Urch (Eds.), pages 53-62 (Humana Press, lnc. 2000)). Random peptide display libraries and kits for screening such libraries are available commercially, for instance from CLONTECH Laboratories, Inc. (Palo Alto, CA), Invitrogen Inc. (San Diego, CA), New England Biolabs, Inc. (Beverly, MA), and Amersham Pharmacia Biotech, Inc. (Piscataway, NJ). Random peptide display libraries can be screened using the receptor sequences disclosed herein to identify proteins, which bind to BCMA or TACI

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2:106 (1991), Courtenay-Luck, "Genetic Manipulation of Monoclonal Antibodies," in Monoclonal Antibodies: Production, Engineering and Clinical Application, Ritter et al. (eds.), page 166 (Cambridge University Press 1995), and Ward et al., "Genetic Manipulation and Expression of Antibodies," in Monoclonal Antibodies: Principles and Applications, Birch et al., (eds.), page 137 (Wiley-Liss, Inc. 1995)).

Another useful anti-receptor antibody is a chimeric antibody. A chimeric antibody comprises the variable domains and complementary determining regions derived from a rodent antibody, while the remainder of the antibody molecule is derived from a human antibody. See, for example, Verma and Boleti, "Engineering Antibody Molecules," in Diagnostic and Therapeutic Antibodies, George and Urch (Eds.), pages 35-52 (Humana Press, Inc. 2000).

Alternatively, an anti-receptor antibody can be derived from a "humanized" monoclonal antibody. Humanized monoclonal antibodies are produced by transferring mouse complementary determining regions from heavy and light variable chains of the mouse immunoglobulin into a human variable domain. Typical residues of human antibodies are then substituted in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi et al., Proc. Nat'l Acad. Sci. USA 86:3833 (1989). Techniques for producing humanized monoclonal antibodies are described, for example, by Jones et al., Nature 321:522 (1986), Carter et al., Proc. Nat'l Acad. Sci. USA 89:4285 (1992), Sandhu, Crit. Rev. Biotech. 12:437 (1992), Singer et al., J. Immun. 150:2844 (1993), Sudhir (ed.), Antibody Engineering Protocols (Humana Press, Inc. 1995), Kelley, "Engineering Therapeutic Antibodies," in Protein Engineering: Principles and Practice, Cleland et al (eds.), pages 399-434 (John Wiley & Sons, Inc. 1996), and by Queen et al., U.S. Patent No. 5,693,762 (1997).

### E. Production of Bispecific Antibodies

As shown in Example 1, the combination of anti-BCMA and anti-TACI antibodies can be advantageous in the treatment of lymphoma. The present invention includes the use of compositions that comprise an antibody component that binds the BCMA extracellular region, and an antibody component that binds the TACI extracellular region. For example, such a "multispecific antibody composition" can comprise a dual reactive anti-BCMA-TACI antibody component, as described above, a heteroantibody mixture (i.e., an aggregate of at least two antibody components, each having a different binding specificity), a bispecific antibody (*i.e.,* an antibody component with two different combining sites), a single chain bispecific polypeptide, and the like.

Bispecific antibodies can be made by a variety of conventional methods. As an illustration, bispecific antibodies have been prepared by oxidative cleavage of Fab' fragments resulting from reductive cleavage of different antibodies. See, for example, Winter et al., Nature 349:293 (1991). This can be carried out by mixing two different F(ab')₂ fragments produced by pepsin digestion of two different antibodies, reductive cleavage to form a mixture of Fab' fragments, followed by oxidative reformation of the disulfide linkages to produce a mixture of F(ab')₂ fragments including bispecific antibodies containing a Fab' potion specific to each of the original epitopes. General techniques for the preparation of such bispecific antibodies can be found, for example, in Nisonhoff et al., Arch Biochem. Biophys. 93:470 (1961), Hammerling et al., J. Exp. Med 128:1461 (1968), and U.S. patent No. 4,331,647.

Alternatively, linkage can be achieved by using a heterobifunctional linker such as maleimide-hydroxysuccinimide ester. Reaction of the ester with an antibody or fragment will derivatize amine groups on the antibody or fragment, and the derivative can then be reacted with, for example, an antibody Fab fragment having free sulfhydryl groups (or, a larger fragment or intact antibody with sulfhydryl groups appended thereto by, for example, Traut's Reagent). Such a linker is less likely to crosslink groups in the same antibody and improves the selectivity of the linkage.

As another example, bispecific F(ab')₂ antibodies can be produced by linking two Fab' fragments via their hinge region SH groups using the bifunctional crosslinker o-phenylenedimaleimide. See, for example, Tso, "F(ab')2 Fusion Proteins and Bispecific F(ab')2," in Chamow and Ashkenazi (Eds.), Antibody Fusion Proteins, pages 127-150 (Wiley-Liss, Inc. 1999), and French, "How to Make Bispecific Antibodies," in George and Urch (Eds.), Diagnostic and Therapeutic Antibodies, pages 333-339 (Humana Press, Inc. 2000).

It is advantageous to link the antibodies or fragments at sites remote from the antigen binding sites. This can be accomplished by, for example, linkage to cleaved interchain sulfydryl groups, as noted above. Another method involves reacting an antibody having an oxidized carbohydrate portion with another antibody which has at lease one free amine function. This results in an initial Schiff base (imine) linkage, which can be stabilized by reduction to a secondary amine, for example, by borohydride reduction, to form the final composite. Such site-specific linkages are disclosed, for small molecules, in U.S. patent No. 4,671,958, and for larger addends in U.S. patent No. 4,699,784.

Alternatively, bispecific antibodies can be produced by fusing two hybridoma cell lines, one cell line that produces anti-BCMA monoclonal antibody, and one cell line that produces anti-TACI monoclonal antibody. Techniques for producing tetradomas are described, for example, by Milstein et al., Nature 305:537 (1983), and Pohl et al., Int. J. Cancer 54:418 (1993).

Bispecific antibodies can also be produced by genetic engineering. For example, vectors containing DNA coding for variable domains of an anti-BCMA monoclonal antibody can be introduced into hybridomas that secrete anti-TACI antibodies. The resulting transfectomas produce bispecific antibodies that bind BCMA and TACI. Alternatively, chimeric genes can be designed that encode both anti-BCMA and anti-TACI binding domains. A variety of genetic strategies for producing bispecifc antibodies are available to those of skill in the art. In one approach, for example, bispecific F(ab')₂ are produced using leucine zippers. See, for example, Tso, "F(ab')2 Fusion Proteins and Bispecific F(ab')2," in Chamow and Ashkenazi (Eds.), Antibody Fusion Proteins, pages 127-150 (Wiley-Liss, Inc. 1999). General techniques for producing bispecific antibodies by genetic engineering are described, for example, by Songsivilai et al., Biochem. Biophys. Res. Commun. 164:271 (1989), Traunecker et al., EMBO J. 10:3655 (1991), and Weiner et al., J. ImmunoL 147:4035 (1991).

A bispecific molecule of the invention can also be a single chain bispecific molecule, such as a single chain bispecific antibody, a single chain bispecific molecule comprising one single chain antibody and a binding determinant, or a single chain bispecific molecule comprising two binding determinants.

Bispecific antibodies can be screened using standard techniques, such as a bispecific ELISA.

### 4. Use of Anti-BCMA-TACI Antibodies to Detect Receptors Proteins

The present invention contemplates the use of anti-BCMA TACI antibodies to screen biological samples *in vitro* for the presence of BCMA and TACI proteins. In one type of *in vitro* assay, antibodies are used in liquid phase. For example, the presence of BCMA or TACI in a biological sample can be tested by mixing the biological sample with a trace amount of labeled BCMA or TACI, and an antibody under conditions that promote binding between the antibody and BCMA or TACI. Complexes of antibody with BCMA or TACI in the sample can be separated from the reaction mixture by contacting the complex with an immobilized protein which binds with the antibody, such as an Fc antibody or *Staphylococcus* protein A. The concentration of BCMA or TACI in the biological sample will be inversely proportional to the amount of labeled BCMA or TACI bound to the antibody and directly related to the amount of free-labeled BCMA or TACI Illustrative biological samples include blood, urine, saliva, tissue biopsy, and autopsy material.

Alternatively, *in vitro* assays can be performed in which an antibody is bound to a solid-phase carrier. For example, antibody can be attached to a polymer, such ' as aminodextran, in order to link the antibody to an insoluble support such as a polymer-coated bead, a plate or a tube. Other suitable *in vitro* assays will be readily apparent to those of skill in the art.

In another approach, antibodies can be used to detect BCMA or TACI in tissue sections prepared from a biopsy specimen. Such immunochemical detection can be used to determine the relative abundance of BCMA or TACI, and to determine the distribution of BCMA or TACI in the examined tissue. General immunochemistry techniques are well established (see, for example, Ponder, "Cell Marking Techniques and Their Application," in Mammalian Development: A Practical Approach, Monk (ed.), pages 115-38 (IRL Press 1987). Coligan at pages 5.8.1-5.8.8, Ausubel (1995) at pages 14.6.1 to 14.6.13 (Wiley Interscience 1990), and Manson (ed.), Methods In Molecular Biology, Vol. 10: Immunochemical Protocols (The Humana Press, Inc. 1992)).

Immunochemical detection can be performed by contacting a biological sample with an antibody, and then contacting the biological sample with a detectably labeled molecule, which binds to the antibody. For example, the detectably labeled molecule can comprise an antibody component that binds to anti-BCMA-TACI antibody. Alternatively, the antibody can be conjugated with avidin/streptavidin (or biotin) and the detectably labeled molecule can comprise biotin (or avidin/streptavidin). Numerous variations of this basic technique are well-known to those of skill in the art.

Alternatively, an antibody can be conjugated with a detectable label to form an immunoconjugate. Suitable detectable labels include, for example, a radioisotope, a fluorescent label, a chemiluminescent label, an enzyme label, a bioluminescent label or colloidal gold. Methods of making and detecting such detectably-labeled immunoconjugates are well-known to those of ordinary skill in the art, and are described in more detail below.

The detectable label can be a radioisotope that is detected by autoradiography. Isotopes that are particularly useful for the purpose of the present invention are ³H, ¹²⁵I, ¹³¹I, ³⁵S and ¹⁴C_{.}

Immunoconjugates can also be labeled with a fluorescent compound. The presence of a fluorescently-labeled antibody is determined by exposing the immunoconjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine.

Alternatively, immunoconjugates can be detectably labeled by coupling an antibody component to a chemiluminescent compound. The presence of the chemiluminescent-tagged immunoconjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester.

Similarly, a bioluminescent compound can be used to label immunoconjugates of the present invention. Bioluminescence is a type of chemiluninescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin.

Alternatively, immunoconjugates can be detectably labeled by linking an anti-BCMA-TACI antibody component to an enzyme. When the antibody-enzyme conjugate is incubated in the presence of the appropriate substrate, the enzyme moiety reacts with the substrate to produce a chemical moiety, which can be detected, for example, by spectrophotometric, fluorometric or visual means. Examples of enzymes that can be used to detectably label polyspecific immunoconjugates include β-galactosidase, glucose oxidase, peroxidase and alkaline phosphatase.

Those of skill in the art will know of other suitable labels, which can be employed in accordance with the present invention. The binding of marker moieties to antibodies can be accomplished using standard techniques known to the art. Typical methodology in this regard is described by Kennedy et al., Clin. Chim. Acta 70:1 (1976), Schurs et al., Clin. Chim. Acta 81:1 (1977), Shih et al., Int'l J. Cancer 46:1101 (1990), Stein et al., Cancer Res. 50:1330 (1990), and Coligan, *supra.*

Moreover, the convenience and versatility of immunochemical detection can be enhanced by using antibodies that have been conjugated with avidin, streptavidin, and biotin (see, for example, Wilchek et al. (eds.), "Avidin-Biotin Technology," Methods In Enzymology, Vol. 184 (Academic Press 1990), and Bayer et al., "Immunochemical Applications of Avidin-Biotin Technology," in Methods In Molecular Biology, Vol. 10, Manson (ed.), pages 149-162 (The Humana Press, Inc. 1992).

Methods for performing immunoassays are well-established. See, for example, Cook and Self, "Monoclonal Antibodies in Diagnostic Immunoassays," in Monoclonal Antibodies: Production, Engineering, and Clinical Application, Ritter and Ladyman (eds.), pages 180-208, (Cambridge University Press, 1995), Perry, "The Role of Monoclonal Antibodies in the Advancement of Immunoassay Technology," in Monoclonal Antibodies: Principles and Applications, Birch and Lennox (eds.), pages 107-120 (Wiley-Liss, Inc. 1995), and Diamandis, Immunoassay (Academic Press, Inc. 1996).

The present invention contemplates compositions comprising an antibody component described herein. Such compositions can further comprise a carrier. The carrier can be a conventional organic or inorganic carrier. Examples of carriers include water, buffer solution, alcohol, propylene glycol, macrogol, sesame oil, corn oil, and the like.

The present invention also contemplates kits for performing an immunological diagnostic assay for BCMA or TACI gene expression. Such kits comprise at least one container comprising an anti-BCMA-TACI antibody, or antibody fragment. A kit may also comprise a second container comprising one or more reagents capable of indicating the presence of BCMA-TACI antibody or antibody fragments. Examples of such indicator reagents include detectable labels such as a radioactive label, a fluorescent label, a chemiluminescent label, an enzyme label, a bioluminescent label, colloidal gold, and the like. A kit may also comprise a means for conveying to the user that BCMA-TACI antibodies or antibody fragments are used to detect BCMA or TACI protein. For example, written instructions may state that the enclosed antibody or antibody fragment can be used to detect BCMA or TACI. The written material can be applied directly to a container, or the written material can be provided in the form of a packaging insert.

### 5. Preparation of Immunoconjugates and Fusion Proteins

The present invention contemplates the use of naked anti-BCMA--TACI antibodies (or naked antibody fragments thereof), as well as the use of immunoconjugates to effect treatment of various disorders, including B-cell malignancies. Immunoconjugates can be prepared using standard techniques. For example, immunoconjugates can be produced by indirectly conjugating a therapeutic agent to an antibody component (see, for example, Shih et al., Int. J: Cancer 41:832-839 (1988); Shih et al., Int. J. Cancer 46:1101-1106 (1990); and Shih et al., U.S. patent No. 5,057,313). Briefly, one standard approach involves reacting an antibody component having an oxidized carbohydrate portion with a carrier polymer that has at least one free amine function and that is loaded with a plurality of drug, toxin, chelator, boron addends, or other therapeutic agent. This reaction results in an initial Schiff base (imine) linkage, which can be stabilized by reduction to a secondary amine to form the final conjugate.

The carrier polymer can be an aminodextran or polypeptide of at least 50 amino acid residues, although other substantially equivalent polymer carriers can also be used. Preferably, the final immunoconjugate is soluble in an aqueous solution, such as mammalian serum, for ease of administration and effective targeting for use in therapy. Thus, solubilizing functions on the carrier polymer will enhance the serum solubility of the final immunoconjugate.

In an alternative approach for producing immunoconjugates comprising a polypeptide therapeutic agent, the therapeutic agent is coupled to aminodextran by glutaraldehyde condensation or by reaction of activated carboxyl groups on the polypeptide with amines on the aminodextran. Chelators can be attached to an antibody component to prepare immunoconjugates comprising radiometals or magnetic resonance enhancers. Illustrative chelators include derivatives of ethylenediaminetetraacetic acid and diethylenetriaminepentaacetic acid. Boron addends, such as carboranes, can be attached to antibody components by conventional methods.

Immunoconjugates can also be prepared by directly conjugating an antibody component with a therapeutic agent. The general procedure is analogous to the indirect method of conjugation except that a therapeutic agent is directly attached to an oxidized antibody component.

As a further illustration, a therapeutic agent can be attached at the hinge region of a reduced antibody component via disulfide bond formation. For example, the tetanus toxoid peptides can be constructed with a single cysteine residue that is used to attach the peptide to an antibody component. As an alternative, such peptides can be attached to the antibody component using a heterobifunctional cross-linker, such as N-succinyl 3-(2-pyridyldithio)proprionate. Yu et al., Int. J. Cancer 56:244 (1994). General techniques for such conjugation are well-known in the art. See, for example, Wong, Chemistry Of Protein Conjugation And Cross-Linking (CRC Press 1991); Upeslacis et al., "Modification of Antibodies by Chemical Methods," in Monoclonal Antibodies: Principles And Applications, Birch et al. (eds.), pages 187-230 (Wiley-Liss, Inc. 1995); Price, "Production and Characterization of Synthetic Peptide-Derived Antibodies," in Monoclonal Antibodies: Production, Engineering And Clinical Application, Ritter et al. (eds.), pages 60-84 (Cambridge University Press 1995).

As described above, carbohydrate moieties in the Fc region of an antibody can be used to conjugate a therapeutic agent. However, the Fc region is absent if an antibody fragment is used as the antibody component of the immunoconjugate. Nevertheless, it is possible to introduce a carbohydrate moiety into the light chain variable region of an antibody or antibody fragment. See, for example, Leung et al., J. Immunol. 154:5919 (1995); Hansen et al., U.S. patent No. 5,443,953 (1995). The engineered carbohydrate moiety is then used to attach a therapeutic agent.

In addition, those of skill in the art will recognize numerous possible variations of the conjugation methods. For example, the carbohydrate moiety can be used to attach polyethyleneglycol in order to extend the half-life of an intact antibody, or antigen-binding fragment thereof, in blood, lymph, or other extracellular fluids. Moreover, it is possible to construct a divalent immunoconjugate by attaching therapeutic agents to a carbohydrate moiety and to a free sulfhydryl group. Such a free sulfhydryl group may be located in the hinge region of the antibody component.

One type of immunoconjugate comprises an antibody component and a polypeptide cytotoxin. An example of a suitable polypeptide cytotoxin is a ribosome-inactivating protein. Type I ribosome-inactivating proteins are single-chain proteins, while type II ribosome-inactivating proteins consist of two nonidentical subunits (A and B chains) joined by a disulfide bond (for a review, see Soria et al., Targeted Diagn. Ther. 7:193 (1992)). Useful type I ribosome-inactivating proteins include polypeptides from *Saponaria officinalis* (*e.g.,* saporin-1, saporin-2, saporin-3, saporin-6), *Momordica charantia (e.g,* momordin), *Byronia dioica* (*e.g.,* bryodin, bryodin-2), *Trichosanthes kirilowii (e.g.,* trichosanthin, trichokirin), *Gelonium multiflorum (e.g.,* gelonin), *Phytolacca americana (e.g.,* pokeweed antiviral protein, pokeweed antiviral protein-II, pokeweed antiviral protein-S), *Phytolacca dodecandra (e.g.,* dodecandrin, *Mirabilis* antiviral protein), and the like. Ribosome-inactivating proteins are described, for example, by Walsh et al., U.S. Patent No. 5,635,384.

Suitable type II ribosome-inactivating proteins include polypeptides from *Ricinus communis (e.g.,* ricin), *Abrus precatorius* (*e.g.,* abrin), *Adenia digitata (e.g.,* modeccin), and the like. Since type II ribosome-inactiving proteins include a B chain that binds galactosides and a toxic A chain that depurinates adensoine, type II ribosome-inactivating protein conjugates should include the A chain. Additional useful ribosome-inactivating proteins include bouganin, clavin, maize ribosome-inactivating proteins, *Vaccaria pyramidata* ribosome-inactivating proteins, nigrine b, basic nigrine 1, ebuline, racemosine b, luffin-a, luffin-b, luffin-S, and other ribosome-inactivating proteins known to those of skill in the art. See, for example, Bolognesi and Stirpe, international publication No. WO98/55623, Colnaghi *et al.,* international publication No. WO97/49726, Hey et al., U.S. Patent No. 5,635,384, Bolognesi and Stirpe, international publication No. WO95/07297, Arias *et al.,* international publication No. WO94/20540 Watanabe et al., J. Biochem. 106:6 977 (1989); Islam et al., Agric. Biol. Chem. 55:229 (1991), and Gao et al., FEBS Lett. 347:257 (1994).

Analogs and variants of naturally-occurring ribosome-inactivating proteins are also suitable for the targeting compositions described herein, and such proteins are known to those of skill in the art. Ribosome-inactivating proteins can be produced using publicly available amino acid and nucleotide sequences. As an illustration, a nucleotide sequence encoding saporin-6 is disclosed by Lorenzetti et al., U.S. Patent No. 5,529,932, while Walsh et al., U.S. Patent No. 5,635,384, describe maize and barley ribosome-inactivating protein nucleotide and amino acid sequences. Moreover, ribosome-inactivating proteins are also commercially available.

Additional polypeptide cytotoxins include ribonuclease, DNase I, *Staphylococcal* enterotoxin-A, diphtheria toxin, *Pseudomonas* exotoxin, and *Pseudomonas* endotoxin. See, for example, Pastan et al., Cell 47:641 (1986), and Goldenberg, CA - A Cancer Journal for Clinicians 44:43 (1994).

Another general type of useful cytotoxin is a tyrosine kinase inhibitor. Since the activation of proliferation by tyrosine kinases has been suggested to play a role in the development and progression of tumors, this activation can be inhibited by anti-BCMA-TACI antibody components that deliver tyrosine kinase inhibitors. Suitable tyrosine kinase inhibitors include isoflavones, such as genistein (5, 7, 4'-trihydroxyisoflavone), daidzein (7,4-dihydroxyisoflavone), and biochanin A (4-methoxygenistein), and the like. Methods of conjugating tyrosine inhibitors to a growth factor are described, for example, by Uckun, U.S. Patent No. 5,911,995.

Another group of useful polypeptide cytotoxins includes immunomodulators. As used herein, the term "immunomodulator" includes cytokines, stem cell growth factors, lymphotoxins, co-stimulatory molecules, hematopoietic factors, and the like, as well as synthetic analogs of these molecules. Examples of immunomodulators include tumor necrosis factor, interleukins *(e.g.,* interieukin-1 (IL-1), IL-2, IL-3, IL-4, IL-5, IL-6, IL-7, IL-8, IL-9, IL- 10, IL-11, IL-12, IL-13, IL-14, IL-15, IL-16, IL-17, IL-18, IL-19, IL-20, and IL-21), colony stimulating factors (e.g., granulocyte-colony stimulating factor and granulocyte macrophage-colony stimulating factor), interferons (e.g., interferons-α -β, -y, -ω, -ε, and -τ), the stem cell growth factor designated "S1 factor," erythropoietin, and thrombopoietin. Illustrative immunomodulator moieties include IL-2, IL-6, IL-10, interferon-γ, TNF-α, and the like.

Immunoconjugates that include an immunomodulator provide a means to deliver an immunomodulator to a target cell, and are particularly useful against tumor cells. The cytotoxic effects of immunomodulators are well known to those of skill in the art. See, for example Klegerman et al., "Lymphokines and Monokines," in Biotechnology And Pharmacy, Pessuto et al. (eds.), pages 53-70 (Chapman & Hall 1993). As an illustration, interferons can inhibit cell proliferation by inducing increased expression of class I histocompatibility antigens on the surface of various cells and thus, enhance the rate of destruction of cells by cytotoxic T lymphocytes. Furthermore, tumor necrosis factors, such as tumor necrosis factor-α, are believed to produce cytotoxic effects by inducing DNA fragmentation.

The present invention also includes immunocongugates that comprise a nucleic acid molecule encoding a cytotoxin. As an example of this approach, Hoganson et al., Human Gene Ther. 9:2565 (1998), describe FGF-2 mediated delivery of a saporin gene by producing an FGF-2-polylysine conjugate which was condensed with an expression vector comprising a saporin gene.

Other suitable toxins are known to those of skill in the art.

Conjugates of cytotoxic polypeptides and antibody components can be prepared using standard techniques for conjugating polypeptides. For example, Lam and Kelleher, U.S. Patent No. 5,055,291, describe the production of antibodies conjugated with either diphtheria toxin fragment A or ricin toxin. The general approach is also illustrated by methods of conjugating fibroblast growth factor with saporin, as described by Lappi et al., Biochem. Biophys. Res. Commun. 160:917 (1989), Soria et al., Targeted Diagn. Ther. 7:193 (1992), Buechler et al., Eur J. Biochem. 234:706 (1995), Behar-Cohen et al., Invest. Ophthalmol. Vis. Sci. 36:2434 (1995), Lappi and Baird, U.S. Patent No. 5,191,067, Calabresi et al., U.S. Patent No. 5,478,804, and Lappi and Baird, U.S. Patent No. 5,576,288. Also see, Ghetie and Vitteta, "Chemical Construction of Immunotoxins," in Drug Targeting: Strategies, Principles, and Applications, Francis and Delgado (Eds.), pages 1-26 (Humana Press, Inc. 2000), Hall (Ed.), Immunotoxin Methods and Protocols (Humana Press, Inc. 2000), and Newton and Rybak, "Construction of Ribonuclease-Antibody Conjugates for Selective Cytotoxicity," in Drug Targeting: Strategies, Principles, and Applications, Francis and Delgado (Eds.), pages 27-35 (Humana Press, Inc. 2000).

Alternatively, fusion proteins comprising an antibody component and a cytotoxic polypeptide can be produced using standard methods. Methods of preparing fusion proteins comprising a cytotoxic polypeptide moiety are well-known in the art of antibody-toxin fusion protein production. For example, antibody fusion proteins comprising an interleukin-2 moiety are described by Boleti et al., Ann. OncoL 6:945 (1995), Nicolet et al., Cancer Gene Ther. 2:161 (1995), Becker et al., Proc. Nat'l Acad. Sci. USA 93:7826 (1996), Hank et al., Clin. Cancer Res. 2:1951 (1996), and Hu et al., Cancer Res. 56:4998 (1996). In addition, Yang et al., Hum. Antibodies Hybridomas 6:129 (1995), describe a fusion protein that includes an F(ab')₂ fragment and a tumor necrosis factor alpha moiety. *Antibody-Pseudofnonas* exotoxin A fusion proteins have been described by Chaudhary et al., Nature 339:394 (1989), Brinkmann et al., Proc. Nat'l Acad. Sci. USA 88:8616 (1991), Batra et al., Proc. Nat'l Acad. Sci. USA 89:5867 (1992), Friedman et al., J. Immunol. 150:3054 (1993), Wels et al., Int. J. Can. 60:137 (1995), Fominaya et al., J. Biol. Chem. 271:10560 (1996), Kuan et al., Biochemistry 35:2872 (1996), and Schmidt et al., Int. J. Can. 65:538 (1996). Antibody-toxin fusion proteins containing a diphtheria toxin moiety have been described by Kreitman et al., Leukemia 7:553 (1993), Nicholls et al., J. Biol. Chem. 268:5302 (1993), Thompson et al., J. Biol. Chem. 270:28037 (1995), and Vallera et al., Blood 88:2342 (1996). Deonarain et al., Tumor Targeting 1:177 (1995), have described an antibody-toxin fusion protein having an RNase moiety, while Linardou et al., Cell Biophys. 24-25:243 (1994), produced an antibody-toxin fusion protein comprising a DNase I component. Gelonin was used as the toxin moiety in the antibody-toxin fusion protein of Better et al., J. Biol. Chem. 270:14951 (1995). As a further example, Dohlsten et al., Proc. Nat'l Acad. Sci. USA 91:8945 (1994), reported an antibody-toxin fusion protein comprising *Staphylococcal* enterotoxin-A. Also see, Newton and Rybak, "Preparation of Recombinant RNase Single-Chain Antibody Fusion Proteins," in Drug Targeting: , Strategies, Principles, and Applications, Francis and Delgado (Eds.), pages 77-95 (Humana Press, Inc. 2000).

As an alternative to a polypeptide cytotoxin, immunoconjugates can comprise a radioisotope as the cytotoxic moiety. For example, an immunoconjugate can comprise an anti-BCMA-TACI antibody component and an α-emitting radioisotope, a β-emitting radioisotope, a γ- emitting radioisotope, an Auger electron emitter, a neutron capturing agent that emits α-particles or a radioisotope that decays by electron capture. Suitable radioisotopes include ¹⁹⁸Au, ¹⁹⁹Au, ³²P, ³³P, ¹²⁵I, ¹³¹I ¹²³L ⁹⁰Y, ¹⁸⁶Re, ¹⁸⁸Re, ⁶⁷Cu, ²¹¹At, ⁴⁷Sc, ¹⁰³Pb, ¹⁰⁹Pd, ²¹²Pb, ⁷¹Ge, ⁷⁷As¹⁰⁵Rh, ¹¹³Ag, ¹¹⁹Sb , ¹²¹Sn, ¹³¹Cs, ¹⁴³Pr, ¹⁶¹Tb, ¹⁷⁷Lu, ¹⁹¹Os, ^{193*M*}Pt, ¹⁹⁷Hg, and the like.

A radioisotope can be attached to an antibody component directly or indirectly, via a chelating agent. For example, ⁶⁷Cu, which provides β-particles and γ-rays, can be conjugated to an antibody component using the chelating agent, p-bromoacetamido-benzyl-tetraethylaminetetraacetic acid. Chase and Shapiro, "Medical Applications of Radioisotopes," in Gennaro (Ed.), Remington: The Science and Practice of Pharmacy, 19th Edition, pages 843-865 (Mack Publishing Company 1995). As an alternative, ⁹⁰Y, which emits an energetic β-particle, can be coupled to an antibody component using diethylenetriaminepentaacetic acid. Moreover, an exemplary suitable method for the direct radiolabeling of an antibody component with ¹³¹I is described by Stein et al., Antibody Immunoconj. Radiopharm. 4:703 (1991). Alternatively, boron addends such as carboranes can be attached to antibody components, using standard techniques.

Another type of suitable cytotoxin for the preparation of immunoconjugates is a chemotherapeutic drug. Illustrative chemotherapeutic drugs include nitrogen mustards, alkyl sulfonates, nitrosoureas, triazenes, folic acid analogs, pyrimidine analogs, purine analogs, antibiotics, epipodophyllotoxins, platinum coordination complexes, and the like. Specific examples of chemotherapeutic drugs include methotrexate, doxorubicin, daunorubicin, cytosinarabinoside, *cis*-platin, vindesine, mitomycin, bleomycin, melphalan, chlorambucil, maytansinoids, calicheamicin, taxol, and the like. Suitable chemotherapeutic agents are described in Remington: The Science and Practice of Pharmacy, 19th Edition (Mack Publishing Co. 1995), and in Goodman And Gilman's The Pharmacological Basis Of Therapeutics, 9th Ed. (MacMillan Publishing Co. 1995). Other suitable chemotherapeutic agents are known to those of skill in the art.

In another approach, immunoconjugates are prepared by conjugating photoactive agents or dyes to an antibody component. Fluorescent and other chromogens, or dyes, such as porphyrins sensitive to visible light, have been used to detect and to treat lesions by directing the suitable light to the lesion. This type of "photoradiation," "phototherapy," or "photodynamic" therapy is described, for example, by Mew et al., J. Immunol. 130:1473 (1983), Jori et al. (eds.), Photodynamic Therapy Of Tumors And Other Diseases (Libreria Progetto 1985), Oseroff et al., Proc. Natl. Acad. Sci. USA 83:8744 (1986), van den Bergh, Chem. Britain 22:430 (1986), Hasan et al., Prog. Clin. Biol. Res. 258:471 (1989), Tatsuta et al., Lasers Surg. Med 9:422 (1989), and Pelegrin et al., Cancer 67:2529 (1991).

The approaches described above can also be used to prepare multispecific antibody compositions that comprise an immunoconjugate.

### 6. Therapeutic Uses of Anti-BCMA-TACI Antibodies

Anti-BCMA-TACI antibodies and multispecific antibody compositions can be used to modulate the immune system by preventing the binding of ZTNF4 with endogenous BCMA and TACI receptors. Such antibodies can be administered to any subject in need of treatment, and the present invention contemplates both veterinary and human therapeutic uses. Illustrative subjects include mammalian subjects, such as farm animals, domestic animals, and human patients.

Multispecific antibody compositions and dual reactive antibodies that bind both BCMA and TACI can be used for the treatment of autoimmune diseases, B cell cancers, immunomodulation, and other pathologies (e.g., TTCP, T cell-mediated diseases, cattleman's disease, autoimmune disease, myelodysplastic syndrome, and the like), renal diseases, graft rejection, and graft versus host disease. The antibodies of the present invention can be targeted to specifically regulate B cell responses during the immune response. Additionally, the antibodies of the present invention can be used to modulate B cell development, antigen presentation by B cells, antibody production, and cytokine production.

Antagonistic anti-BCMA-TACI antibodies can be useful to neutralize the effects of ZrNF4 for treating B cell lymphomas and leukemias, chronic or acute-lymphocytic leukemia, myelomas such as multiple myeloma, plasma cytomas, and lymphomas such as non-Hodgkins lymphoma, for which an increase in ZTNF4 polypeptides is associated, or where ZTNF4 is a survival factor or growth factor. Anti-BCMA-TACI antibodies can also be used to treat Epstein Barr virus-associated lymphomas arising in immunocompromised patients (e.g., AIDS or organ transplant).

Multiple myeloma, a B-cell malignancy with mature plasma cell morphology, is characterized by the neoplastic transformation of a single clone of plasma cells. These plasma cells proliferate in bone marrow and may invade adjacent bone. Variant forms of multiple myeloma include overt multiple myeloma, smoldering multiple myeloma, plasma cell leukemia, non-secretory myeloma, IgD myeloma, osteosclerotic myeloma, solitary plasmacytoma of bone, and extramedullary plasmacytoma (see, for example, Braunwald, et al. (eds), Harrison's Principles of Internal Medicine, 15th Edition (McGraw-Hill 2001)). As described in Example 1, treatment with anti-BCMA-TACI antibodies prolonged survival in a murine model of multiple myeloma. Accordingly, the present invention includes the use of anti-BCMA-TACI antibodies to treat multiple myeloma and its variant forms, such as plasma cell leukemia. More generally, anti-BCMA-TACI antibodies are useful for targeting disorders characterized by mature B cells.

Anti-BCMA-TACI antibodies that induce a signal by binding with BCMA or TACI may inhibit the growth of lymphoma and leukemia cells directly via induction of signals that lead to growth inhibition, cell cycle arrest, apoptosis, or tumor cell death. BCMA-TACI antibodies that initiate a signal are preferred antibodies to directly inhibit or kill cancer cells. In addition, agonistic anti-BCMA-TACI monoclonal antibodies may activate normal B cells and promote an anticancer immune response. Anti-BCMA-TACI antibodies may directly inhibit the growth of leukemias, lymphomas, and multiple myelomas, and the antibodies may engage immune effector functions. Anti-BCMA-TACI monoclonal antibodies may enable antibody-dependent cellular cytotoxicity, complement dependent cytotoxicity, and phagocytosis.

ZTNF4 is expressed in neutrophils, monocytes, dendritic cells, and activated monocytes. In certain autoimmune disorders (e.g., myasthenia gravis, and rheumatoid arthritis), B cells might exacerbate autoimmunity after activation by ZTNF4. Immunosuppressant proteins that selectively block the action of B-lymphocytes would be of use in treating disease. Autoantibody production is common to several autoimmune diseases and contributes to tissue destruction and exacerbation of disease. Autoantibodies can also lead to the occurrence of immune complex deposition complications and lead to many symptoms of systemic lupus erythematosus, including kidney failure, neuralgic symptoms and death. Modulating antibody production independent of cellular response would also be beneficial in many disease states. B cells have also been shown to play a role in the secretion of arthritogenic immunoglobulins in rheumatoid arthritis. As such, inhibition of ZTNF4 antibody production would be beneficial in treatment of autoimmune diseases such as myasthenia gravis and rheumatoid arthritis. Immunosuppressant therapeutics such as anti-BCMA-TACI antibodies that selectively block or neutralize the action of B-lymphocytes would be useful for such purposes.

The invention provides methods employing anti-BCMA-TACI antibodies, or multispecific antibody compositions, for selectively blocking or neutralizing the actions of B-cells in association with end stage renal diseases, which may or may not be associated with autoimmune diseases. Such methods would also be useful for treating immunologic renal diseases. Such methods would be would be useful for treating glomerulonephritis associated with diseases such as membranous nephropathy, IgA nephropathy or Berger's Disease, IgM nephropathy, Goodpasture's Disease, post-infectious glomerulonephritis, mesangioproliferative disease, chronic lymphocytic leukemia, minimal-change nephrotic syndrome. Such methods would also serve as therapeutic applications for treating secondary glomerulonephritis or vasculitis associated with such diseases as lupus, polyarteritis, Henoch-Schonlein, Scleroderma, HIV-related diseases, amyloidosis or hemolytic uremic syndrome. The methods of the present invention would also be useful as part of a therapeutic application for treating interstitial nephritis or pyelonephritis associated with chronic pyelonephritis, analgesic abuse, nephrocalcinosis, nephropathy caused by other agents, nephrolithiasis, or chronic or acute interstitial nephritis.

The present invention also provides methods for treatment of renal or urological neoplasms, multiple myelomas, lymphomas, leukemias, light chain neuropathy, or amyloidosis.

The invention also provides methods for blocking or inhibiting activated B cells using anti-BCMA-TACI antibodies, or multispecific antibody compositions, for the treatment of asthma and other chronic airway diseases such as bronchitis and emphysema.

Also provided are methods for inhibiting or neutralizing a T cell response using anti-BCMA-TACI antibodies, or multispecific antibody compositions, for immunosuppression, in particular for such therapeutic use as for graft-versus-host disease and graft rejection. Moreover, anti-BCMA-TACI antibodies, or multispecific antibody compositions, would be useful in therapeutic protocols for treatment of such autoimmune diseases as insulin dependent diabetes mellitus (IDDM), multiple sclerosis, rheumatoid arthritis, systemic lupus erythematosus, inflammatory bowel disease (IBD), and Crohn's Disease. Methods of the present invention would have additional therapeutic value for treating chronic inflammatory diseases, in particular to lessen joint pain, swelling, anemia and other associated symptoms as well as treating septic shock.

B cell responses are important in fighting infectious diseases including bacterial, viral, protozoan and parasitic infections. Antibodies against infectious microorganisms can immobilize the pathogen by binding to antigen followed by complement mediated lysis or cell mediated attack. Agonistic, or signaling, anti-BCMA-TACI antibodies may serve to boost the humoral response and would be a useful therapeutic for individuals at risk for an infectious disease or as a supplement to vaccination.

Well established animal models are available to test *in vivo* efficacy of anti-BCMA-TACI antibodies, or multispecific antibody compositions, of the present invention in certain disease states. As an illustration, anti-BCMA-TACI antibodies can be tested in *vivo* in a number of animal models of autoimmune disease, such as MRL-*lprllpr* or NZB x NZW F1 congenic mouse strains which serve as a model of systemic lupus erythematosus. Such animal models are known in the art.

Offspring of a cross between New Zealand Black (NZB) and New Zealand White (NZW) mice develop a spontaneous form of systemic lupus erythematosus that closely resembles systemic lupus erythematosus in humans. The offspring mice, known as NZBW begin to develop IgM autoantibodies against T-cells at one month of age, and by 5-7 months of age, Ig anti-DNA autoantibodies are the dominant immunoglobulin. Polyclonal B-cell hyperactivity leads to overproduction of autoantibodies. The deposition of these autoantibodies, particularly ones directed against single stranded DNA is associated with the development of glomerulonephritis, which manifests clinically as proteinuria, azotemia, and death from renal failure. Kidney failure is the leading cause of death in mice affected with spontaneous systemic lupus erythematosus, and in the NZBW strain, this process is chronic and obliterative. The disease is more rapid and severe in females than males, with mean survival of only 245 days as compared to 406 days for the males. While many of the female mice will be symptomatic (proteinuria) by 7-9 months of age, some can be much younger or older when they develop symptoms. The fatal immune nephritis seen in the NZBW mice is very similar to the glomerulonephritis seen in human systemic lupus erythematosus, making this spontaneous murine model useful for testing of potential systemic lupus erythematosus therapeutics.

Murine models of experimental allergic encephalomyelitis have been used as tools to investigate both the mechanisms of immune-mediated disease, and methods of potential therapeutic intervention. The model resembles human multiple sclerosis, and produces demyelination as a result of T-cell activation to neural proteins such as myelin basic protein, or proteolipid protein. Inoculation with antigen leads to induction of CD4+, class II MHC-restricted T-cells. Changes in the protocol for experimental allergic encephalomyelitis can produce acute, chronic-relapsing, or passive-transfer variants of the model.

In the collagen-induced arthritis model, mice develop chronic inflammatory arthritis, which closely resembles human rheumatoid arthritis. Since collagen-induced arthritis shares similar immunological and pathological features with rheumatoid arthritis, this makes it an ideal model for screening potential human antiinflammatory compounds. Another advantage in using the collagen-induced arthritis model is that the mechanisms of pathogenesis are known. The T and B cell epitopes on type II collagen have been identified, and various immunological (delayed-type hypersensitivity and anti-collagen antibody) and inflammatory (cytokines, chemokines, and matrix-degrading enzymes) parameters relating to immune-mediating arthritis have been determined, and can be used to assess test compound efficacy in the models.

Myasthenia gravis is another autoimmune disease for which murine models are available. Myasthenia gravis is a disorder of neuromuscular transmission involving the production of autoantibodies directed against the nicotinic acetylcholine receptor. Myasthenia gravis is acquired or inherited with clinical features including abnormal weakness and fatigue on exertion. A mouse model of myasthenia gravis have been established. Experimental autoimmune myasthenia gravis is an antibody mediated disease characterized by the presence of antibodies to acetylcholine receptor. These antibodies destroy the receptor leading to defective neuromuscular electrical impulses, resulting in muscle weakness. In the experimental autoimmune myasthenia gravis model, mice are immunized with the nicotinic acetylcholine receptor. Clinical signs of myasthenia gravis become evident weeks after the second immunization. Experimental autoimmune myasthenia gravis is evaluated by several methods including measuring serum levels of acetylcholine receptor antibodies by radioimmunoassay, measuring muscle acetylcholine receptor, or electromyography.

Generally, the dosage of administered anti-BCMA-TACI antibodies, or multispecific antibody compositions, will vary depending upon such factors as the subject's age, weight, height, sex, general medical condition and previous medical history. As an illustration, anti-BCMA-TACI antibodies, or multispecific antibody compositions, can be administered at low protein doses, such as 20 to 100 milligrams protein per dose, given once, or repeatedly. Alternatively, anti-BCMA-TACI antibodies, or multispecific antibody compositions, can be administered in doses of 30 to 90 milligrams protein per dose, or 40 to 80 milligrams protein per dose, or 50 to 70 milligrams protein per dose, although a lower or higher dosage also may be administered as circumstances dictate.

Administration of antibody components to a subject can be intravenous, intraarterial, intraperitoneal, intramuscular, subcutaneous, intrapleural, intrathecal, by perfusion through a regional catheter, or by direct intralesional injection. When administering therapeutic proteins by injection, the administration may be by continuous infusion or by single or multiple boluses. Additional routes of administration include oral, mucosal-membrane, pulmonary, and transcutaneous.

A pharmaceutical composition comprising an anti-BCMA-TACI antibody, or bispecific antibody components, can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. Sterile phosphate-buffered saline is one example of a pharmaceutically acceptable carrier. Other suitable carriers are well-known to those in the art. See, for example, Gennaro (ed.), Remington's Pharmaceutical Sciences, 19th Edition (Mack Publishing Company 1995).

For purposes of therapy, anti-BCMA-TACI antibodies, or bispecific antibody components, and a pharmaceutically acceptable carrier, are administered to a patient in a therapeutically effective amount. A combination of anti-BCMA-TACI antibodies, or bispecific antibody components, and a pharmaceutically acceptable carrier is said to be administered in a "therapeutically effective amount" if the amount administered is physiologically significant. An agent is physiologically significant if its presence results in a detectable change in the physiology of a recipient patient. For example, an agent used to treat inflammation is physiologically significant if its presence alleviates the inflammatory response. As another example, an agent used to inhibit the growth of tumor cells is physiologically significant if the administration of the agent results in a decrease in the number of tumor cells, decreased metastasis, a decrease in the size of a solid tumor, or increased necrosis of a tumor.

A pharmaceutical composition comprising anti-BCMA-TACI antibodies, or bispecific antibody components, can be furnished in liquid form, in an aerosol, or in solid form. Liquid forms, are illustrated by injectable solutions and oral suspensions. Exemplary solid forms include capsules, tablets, and controlled-release forms. The latter form is illustrated by miniosmotic pumps and implants (Bremer et al., Pharm. Biotechnol. 10:239 (1997); Ranade, "Implants in Drug Delivery," in Drug Delivery Systems, Ranade and Hollinger (eds.), pages 95-123 (CRC Press 1995); Bremer et al., "Protein Delivery with Infusion Pumps," in Protein Delivery: Physical Systems, Sanders and Hendren (eds.), pages 239-254 (Plenum Press 1997); Yewey et al., "Delivery of Proteins from a Controlled Release Injectable Implant," in Protein Delivery: Physical Systems, Sanders and Hendren (eds.), pages 93-117 (Plenum Press 1997)).

Those of skill in the art can devise various pharmaceutical compositions using standard techniques. See, for example, Lieberman et al., (Eds.), Pharmaceutical Dosage Forms: Tablets, Vol. 1, 2nd Edition (Marcel Dekker, Inc. 1989), Lieberman et al., (Eds.), Pharmaceutical Dosage Forms. Tablets, Vol. 2, 2nd Edition (Marcel Dekker, Inc. 1990), Lieberman et al., (Eds.), Pharmaceutical Dosage Forms: Tablets, Vol. 3, 2nd Edition (Marcel Dekker, Inc. 1990), Lieberman et al., (Eds.), Pharmaceutical Dosage Forms: Disperse Systems, Vol. 1, 2nd Edition (Marcel Dekker, Inc. 1996), Lieberman et al., (Eds.), Pharmaceutical Dosage Forms: Disperse Systems, Vol. 2, 2nd Edition (Marcel Dekker, Inc. 1996), Lieberman et al., (Eds.), Pharmaceutical Dosage Forms: Disperse Systems, Vol. 3, 2nd Edition (Marcel Dekker, Inc. 1998), Avis et al., (Eds.), Pharmaceutical Dosage Forms: Parenteral Medications, Vol. 1, 2nd Edition (Marcel Dekker, Inc. 1991), Lieberman et al., (Eds.), Pharmaceutical Dosage Forms: Parenteral Medications, Vol. 2, 2nd Edition (Marcel Dekker, Inc. 1992), and Avis et al., (Eds.), Pharmaceutical Dosage Forms: Parenteral Medications, Vol. 3, 2nd Edition (Marcel Dekker, Inc. 1993).

As another example, liposomes provide a means to deliver anti-BCMA-TACI antibodies, or bispecific antibody components, to a subject intravenously, intraperitoneally, intrathecally, intramuscularly, subcutaneously, or via oral administration, inhalation, or intranasal administration. Liposomes are microscopic vesicles that consist of one or more lipid bilayers surrounding aqueous compartments (see, generally, Bakker-Woudenberg et al., Eur. J. Clin. Microbiol. Infect. Dis. 12 (Suppl. 1):S61 (1993), Kim, Drugs 46:618 (1993), and Ranade, "Site-Specific Drug Delivery Using Liposomes as Carriers," in Drug Delivery Systems, Ranade and Hollinger (Eds.), pages 3-24 (CRC Press 1995)). Liposomes are similar in composition to cellular membranes and as a result, liposomes can be administered safely and are biodegradable. Depending on the method of preparation, liposomes may be unilamellar or multilamellar, and liposomes can vary in size with diameters ranging from 0.02 µm to greater than 10 µm. A variety of agents can be encapsulated in liposomes: hydrophobic agents partition in the bilayers and hydrophilic agents partition within the inner aqueous space(s) (see, for example, Machy et al., Liposomes In Cell Biology And Pharmacology (John Libbey 1987), and Ostro et al., American J. Hosp. Pharm 46:1576 (1989)). Moreover, it is possible to control the therapeutic availability of the encapsulated agent by varying liposome size, the number of bilayers, lipid composition, as well as the charge and surface characteristics of the liposomes.

Liposomes can adsorb to virtually any type of cell and then slowly release the encapsulated agent. Alternatively, an absorbed liposome may be endocytosed by cells that are phagocytic_{;} Endocytosis is followed by intralysosomal degradation of liposomal lipids and release of the encapsulated agents (Scherphof et al., Ann. N.Y. Acad. Sci. 446:368 (1985)). After intravenous administration, small liposomes (0.1 to 1.0 µm) are typically taken up by cells of the reticuloendothelial system, located principally in the liver and spleen, whereas liposomes larger than 3.0 µm are deposited in the lung. This preferential uptake of smaller liposomes by the cells of the reticuloendothelial system has been used to deliver chemotherapeutic agents to macrophages and to tumors of the liver.

The reticuloendothelial system can be circumvented by several methods including saturation with large doses of liposome particles, or selective macrophage inactivation by pharmacological means (Claassen et al., Biochim. Biophys. Acta 802:428 (1984)). In addition, incorporation of glycolipid- or polyethelene glycol-derivatized phospholipids into liposome membranes has been shown to result in a significantly reduced uptake by the reticuloendothelial system (Allen et al., Biochim. Biophys. Acta 1068:133 (1991); Allen et al., Biochim. Biophys. Acta 1150:9 (1993)).

As an alternative to administering liposomes that comprise an anti-BCMA-TACI antibody component, target cells can be prelabeled with biotinylated anti-BCMA-TACI antibodies. After plasma elimination of free antibody, streptavidin-conjugated liposomes are administered. This general approach is described, for example, by Harasym et al., Adv. Drug Deliv. Rev. 32:99 (1998). Such an approach can also be used to prepare multispecific antibody compositions.

Polypeptides comprising an anti-BCMA-TACI antibody component, or bispecific antibody components, can be encapsulated within liposomes, or attached to the exterior of liposomes, using standard techniques (see, for example, Anderson et al., Infect. Immun. 31:1099 (1981), Wassef et al., Meth. Enzymol. 149:124 (1987), Anderson et al., Cancer Res. 50:1853 (1990), Cohen et al., Biochim. Biophys. Acta 1063:95 (1991), Alving et al. "Preparation and Use of Liposomes in Immunological Studies," in Liposome Technology, 2nd Edition, Vol. III, Gregoriadis (Ed.), page 317 (CRC Press 1993), and Ansell et al., "Antibody Conjugation Methods for Active Targeting of Liposomes," in Drug Targeting: Strategies, Principles, and Applications, Francis and Delgado (Eds.), pages 51-68 (Humana Press, Inc. 2000)). As noted above, therapeutically useful liposomes may contain a variety of components. For example, liposomes may comprise lipid derivatives of poly(ethylene glycol) (Allen et al., Biochim. Biophys. Acta 1150:9 (1993)).

Degradable polymer microspheres have been designed to maintain high systemic levels of therapeutic proteins. Microspheres are prepared from degradable polymers such as poly(lactide-co-glycolide) (PLG), polyanhydrides, poly (ortho esters), nonbiodegradable ethylvinyl acetate polymers, in which proteins are entrapped in the polymer (Gombotz and Pettit, Bioconjugate Chem. 6:332 (1995); Ranade, "Role of Polymers in Drug Delivery," in Drug Delivery Systems, Ranade and Hollinger (eds.), pages 51-93 (CRC Press 1995); Roskos and Maskiewicz, "Degradable Controlled Release Systems Useful for Protein Delivery," in Protein Delivery: Physical Systems, Sanders and Hendren (Eds.), pages 45-92 (Plenum Press 1997); Bartus et al., Science 281:1161 (1998); Putney and Burke, Nature Biotechnology 16:153 (1998); Putney, Curr. Opin. Chem. Biol. 2:548 (1998)). Polyethylene glycol (PEG)-coated nanospheres can also provide carriers for intravenous administration of therapeutic proteins (see, for example, Gref et al., Pharm. Biotechnol. 10:167 (1997)).

The present invention also contemplates chemically modified antibody components, in which an antibody component is linked with a polymer. Typically, the polymer is water soluble so that an antibody component does not precipitate in an aqueous environment, such as a physiological environment. An example of a suitable polymer is one that has been modified to have a single reactive group, such as an active ester for acylation, or an aldehyde for alkylation. In this way, the degree of polymerization can be controlled. An example of a reactive aldehyde is polyethylene glycol propionaldehyde, or mono-(C₁-C₁₀) alkoxy, or aryloxy derivatives thereof (see, for example, Harris, et al., U.S. Patent No. 5,252,714). The polymer may be branched or unbranched. Moreover, a mixture of polymers can be used to produce conjugates with antibody components.

Suitable water-soluble polymers include polyethylene glycol (PEG), monomethoxy-PEG, mono-(C₁-C₁₀)alkoxy-PEG, aryloxy-PEG, poly-(N-vinyl pyrrolidone)PEG, tresyl monomethoxy PEG, PEG propionaldehyde, bis-succinimidyl carbonate PEG, propylene glycol homopolymers, a polypropylene oxide/ethylene oxide co-polymer, polyoxyethylated polyols (e.g.*,* glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A conjugate can also comprise a mixture of such water-soluble polymers.

As an illustration, a polyalkyl oxide moiety can be attached to the *N-*terminus of antibody component. PEG is one suitable polyalkyl oxide. For example, an antibody component can be modified with PEG, a process known as "PEGylation." PEGylation of an antibody component can be carried out by any of the PEGylation reactions known in the art (see, for example, EP 0 154 316, Delgado et al., Critical Reviews in Therapeutic Drug Carrier Systems 9:249 (1992), Duncan and Spreafico, Clin. Pharmacokinet. 27:290 (1994), and Francis et al., Int J Hematol 68: 1 (1998)). For example, PEGylation can be performed by an acylation reaction or by an alkylation reaction with a reactive polyethylene glycol molecule. In an alternative approach, antibody component conjugates are formed by condensing activated PEG, in which a terminal hydroxy or amino group of PEG has been replaced by an activated linker (see, for example, Karasiewicz et al., U.S. Patent No. 5,382,657).

PEGylation by acylation typically requires reacting an active ester derivative of PEG with an antibody component. An example of an activated PEG ester is PEG esterified to *N-*hydroxysuccinimide. As used herein, the term "acylation" includes the following types of linkages between an antibody component and a water soluble polymer: amide, carbamate, urethane, and the like. Methods for preparing PEGylated anti-BCMA-TACI antibody components by acylation will typically comprise the steps of (a) reacting an antibody component with PEG (such as a reactive ester of an aldehyde derivative of PEG) under conditions whereby one or more PEG groups attach to the antibody component, and (b) obtaining the reaction product(s). Generally, the optimal reaction conditions for acylation reactions will be determined based upon known parameters and desired results. For example, the larger the ratio of PEG:antibody component, the greater the percentage of polyPEGylated antibody component product.

The product of PEGylation by acylation is typically a polyPEGylated antibody component product, wherein the lysine ε-amino groups are PEGylated via an acyl linking group. An example of a connecting linkage is an amide. Typically, the resulting antibody component will be at least 95% mono-, di-, or tri-pegylated, although some species with higher degrees of PEGylation may be formed depending upon the reaction conditions. PEGylated species can be separated from unconjugated antibody component using standard purification methods, such as dialysis, ultrafiltration, ion exchange chromatography, affinity chromatography, and the like.

PEGylation by alkylation generally involves reacting a terminal aldehyde derivative of PEG with antibody component in the presence of a reducing agent. PEG groups can be attached to the polypeptide via a -CH₂-NH group.

Derivatization via reductive alkylation to produce a monoPEGylated product takes advantage of the differential reactivity of different types of primary amino groups available for derivatization. Typically, the reaction is performed at a pH that allows one to take advantage of the pKa differences between the ε-amino groups of the lysine residues and the α-amino group of the *N*-terminal residue of the protein. By such selective derivatization, attachment of a water-soluble polymer that contains a reactive group such as an aldehyde, to a protein is controlled. The conjugation with the polymer occurs predominantly at the *N*-terminus of the protein without significant modification of other reactive groups such as the lysine side chain amino groups.

Reductive alkylation to produce a substantially homogenous population of monopolymer antibody component conjugate molecule can comprise the steps of: (a) reacting an antibody component with a reactive PEG under reductive alkylation conditions at a pH suitable to permit selective modification of the α-amino group at the amino terminus of the antibody component, and (b) obtaining the reaction product(s). The reducing agent used for reductive alkylation should be stable in aqueous solution and preferably be able to reduce only the Schiff base formed in the initial process of reductive alkylation. Preferred reducing agents include sodium borohydride, sodium cyanoborohydride, dimethylamine borane, trimethylamine borane, and pyridine borane.

For a substantially homogenous population of monopolymer antibody component conjugates, the reductive alkylation reaction conditions are those which permit the selective attachment of the water soluble polymer moiety to the *N-*terminus of the antibody component. Such reaction conditions generally provide for pKa differences between the lysine amino groups and the α-amino group at the *N*-terminus. The pH also affects the ratio of polymer to protein to be used. In general, if the pH is lower, a larger excess of polymer to protein will be desired because the less reactive the *N*-terminal α-group, the more polymer is needed to achieve optimal conditions. If the pH is higher, the polymer:antibody component need not be as large because more reactive groups are available. Typically, the pH will fall within the range of 3 to 9, or 3 to 6.

General methods for producing conjugates comprising a polypeptide and water-soluble polymer moieties are known in the art. See, for example, Karasiewicz et al., U.S. Patent No. 5,382,657, Greenwald et al., U.S. Patent No. 5,738, 846, Nieforth et al., Clin. Pharmacol. Ther. 59:636 (1996), Monkarsh et al., Anal. Biochem. 247:434 (1997)).

Polypeptide cytotoxins can also be conjugated with a soluble polymer using the above methods either before or after conjugation to an antibody component. Soluble polymers can also be conjugated with antibody fusion proteins.

Naked anti-BCMA-TACI antibodies, or antibody fragments, can be supplemented with immunoconjugate or antibody fusion protein administration. In one variation, naked anti-BCMA-TACI antibodies (or naked antibody fragments) are administered with low-dose radiolabeled anti-BCMA-TACI antibodies or antibody fragments. As a second alternative, naked anti-BCMA-TACI antibodies (or antibody fragments) are administered with low-dose radiolabeled anti-BCMA-TACI antibodies-cytokine immunoconjugates. As a third alternative, naked anti-BCMA-TACI antibodies (or antibody fragments) are administered with anti-BCMA-TACI-cytokine immunoconjugates that are not radiolabeled. With regard to "low doses" of ¹³¹I-labeled immunoconjugates, a preferable dosage is in the range of 15 to 40 mCi, while the most preferable range is 20 to 30 mCi. In contrast, a preferred dosage of ⁹⁰Y-labeled immunoconjugates is in the range from 10 to 30 mCi, while the most preferable range is 10 to 20 mCi. Similarly, bispecific antibody components can be supplemented with immunoconjugate or antibody fusion protein administration.

Immunoconjugates having a boron addend-loaded carrier for thermal neutron activation therapy will normally be effected in similar ways. However, it will be advantageous to wait until non-targeted immunoconjugate clears before neutron irradiation is performed. Clearance can be accelerated using an antibody that binds to the immunoconjugate. See U.S. Pat. No. 4,624,846 for a description of this general principle.

The present invention also contemplates a method of treatment in which immunomodulators are administered to prevent, mitigate or reverse radiation-induced or drug-induced toxicity of normal cells, and especially hematopoietic cells. Adjunct immunomodulator therapy allows the administration of higher doses of cytotoxic agents due to increased tolerance of the recipient mammal. Moreover, adjunct immunomodulator therapy can prevent, palliate, or reverse dose-limiting marrow toxicity. Examples of suitable immunomodulators for adjunct therapy include granulocyte-colony stimulating factor, granulocyte macrophage-colony stimulating factor, thrombopoietin, IL-1, IL-3, IL-12, and the like. The method of adjunct immunomodulator therapy is disclosed by Goldenberg, U.S. Pat. No. 5,120,525.

The efficacy of anti-BCMA-TACI antibody therapy can be enhanced by supplementing naked antibody components with immunoconjugates and other forms of supplemental therapy described herein. In such multimodal regimens, the supplemental therapeutic compositions can be administered before, concurrently or after administration of naked anti-BCMA-TACI antibodies. Multimodal therapies of the present invention further include immunotherapy with naked anti-BCMA-TACI antibody components supplemented with administration of anti-BCMA-TACI immunoconjugates. In another form of multimodal therapy, subjects receive naked anti-BCMA-TACI antibodies and standard cancer chemotherapy.

The antibodies, immunoconjugates, and antibody fusion proteins described herein can also be advantageously supplemented with antibody components (*e.g.*, naked antibodies, naked antibody fragments, immunoconjugates, antibody fusion proteins, *etc.*) that bind the so-called "stalk region" of the TACI receptor, which resides between the second cysteine-rich region and the transmembrane domain. Studies indicate that, to an extent, TACI proteins are cleaved and shed by cells, leaving a small extracellular peptide, or stalk on the cell surface. A murine monoclonal antibody was found to be therapeutically useful in a lymphoma murine model. Epitope mapping indicates that the antibody binds with a fragment of the TACI extracellular domain, represented by amino acid residues 110 to 118 of SEQ ID NO:4. Antibodies can be generated against a polypeptide representing the region between the second cysteine-rich domain and the transmembrane domain (amino acid residues 105 to 166 of SEQ ID NO:4), or to a fragment thereof (e.g., amino acid residues 110 to 118 of SEQ ID NO:4). Such antibodies are particularly useful for treatment of TACI-bearing tumor cells, such as B-lymphoma cells, myeloma cells, and the like.

The antibodies and antibody fragments of the present invention can be used as vaccines to treat the various disorders and diseases described above. As an example, an antibody component of a dual reactive BCMA-TACI monoclonal antibody can provide a suitable basis for a vaccine. Cysteine-rich regions of BCMA and TACI receptors can also provide useful components for a vaccine. For example, a vaccine can comprise at least one of the following polypeptides: a polypeptide comprising amino acid residues 8 to 41 of SEQ ID NO:2, a polypeptide comprising amino acid residues 34 to 66 of SEQ ID NO:4, and a polypeptide comprising amino acid residues 71 to 104 of SEQ ID NO:4.

The efficacy of an antibody component as a vaccine can be enhanced by conjugating the antibody component to a soluble immunogenic carrier protein. Suitable carrier proteins include tetanus toxin/toxoid, NTHi high molecular weight protein, diphtheria toxin/toxoid, detoxified *P. aeruginosa* toxin A, cholera toxin/toxoid, pertussis toxin/toxoid, *Clostridium perfringens* exotoxins/toxoid, hepatitis B surface antigen, hepatitis B core antigen, rotavirus VP 7 protein, respiratory syncytial virus F and G protein, and the like. Methods of preparing conjugated vaccines are known to those of skill in the art. See, for example, Cruse and Lewis (Eds.), Conjugate Vaccines (S. Karger Publishing 1989), and O'Hagan (Ed.), Vaccine Adjuvants (Humana Press, Inc. 2000). A vaccination composition can also include an adjuvant. Examples of suitable adjuvants include aluminum hydroxide and lipid. Methods of formulating vaccine compositions are well-known to those of ordinary skill in the art. See, for example, Rola, "Immunizing Agents and Diagnostic Skin Antigens," in Remington: The Science and Practice of Pharmacy, 19th Edition, Gennaro (Ed.), pages 1417-1433 (Mack Publishing Company 1995).

Pharmaceutical compositions may be supplied as a kit comprising a container that comprises anti-BCMA-TACI antibody components, or bispecific antibody components. Therapeutic molecules can be provided in the form of an injectable solution for single or multiple doses, or as a sterile powder that will be reconstituted before injection. Alternatively, such a kit can include a dry-powder disperser, liquid aerosol generator, or nebulizer for administration of an anti-BCMA-TACI antibody component. Such a kit may further comprise written information on indications and usage of the pharmaceutical composition. Moreover, such information may include a statement that the composition is contraindicated in patients with known hypersensitivity to exogenous antibodies.

The present invention, thus generally described, will be understood more readily by reference to the following examples, which are provided by way of illustration and are not intended to be limiting of the present invention.

### EXAMPLE 1

### Antibody Treatment in Xenogeneic Lymphoma and Multiple Myeloma Models

SCID mice (C.B.-17; Taconic; Germantown, NY) were injected (i.v.) with 10⁶ IM-9 human lymphoma cells to produce a disseminated model of lymphoma, and the mice were treated with monoclonal antibodies 24 hours later. Monoclonal antibodies were administered at a dose of 1 mg/kg every fourth day for a total of five injections. The mice were monitored for weight loss, hind limb paralysis, and morbidity. Groups of six mice were treated with the following antibodies: dual reactive BCMA-TACI monoclonal antibody, 255.7, TACI murine monoclonal antibody 248.24, a combination of dual reactive BCMA-TACI monoclonal antibody 255.7 and TACI murine monoclonal antibody 248.24 (1 mg/kg each), RITUXAN (a chimeric mouse/human anti-CD20 antibody), and a negative control murine monoclonal antibody (238.12).

As shown in Figure 1, the combination of BCMA and TACI monoclonal antibodies was much more effective in prolonging survival than any other monoclonal antibody treatment. All mice treated with both antibodies survived beyond 46 days, while other monoclonal antibody treatments resulted in 67% to 0%o survival at 46 days. The data suggest that targeting both TACI and BCMA simultaneously is superior than therapy with either anti-BCMA or anti-TACI alone.

In another study, SCID mice were injected (i.v.) with RPMI 8226 human multiple myeloma cell line to produce a disseminated model of multiple myeloma, and the mice were treated with monoclonal antibodies. The results indicate that dual reactive BCMA-TACI monoclonal antibody 255.7 prolongs survival in this murine model of multiple myeloma.

### EXAMPLE 2

### Antibody Treatment Lymphoma and Myeloma Cell Lines In Vitro

A human Burkitt's lymphoma cell line, HS Sultan, and a human multiple myeloma cell line, RPMI 8226, were incubated *in vitro* with BCMA monoclonal antibody, TACI monoclonal antibody, and combinations of BCMA plus TACI monoclonal antibodies. Tumor cell lines were cultured 2.5 days with monoclonal antibodies present at 0.375 µg/ml to 6 µg/ml, and then proliferation was assessed by measuring tritiated thymidine incorporation. In this study, the monoclonal antibodies were: (1) BCMA-TACI antibody (255.7), (2) BCMA antibody (255.4), (3) TACI antibody (248.24), (4) BCMA-TACI antibody (255.7) plus TACI antibody (248.24), (5) CD20 antibody (1F5), and (6) negative control murine IgG2a.

Although the anti-CD20 monoclonal antibody was found to be the most effective treatment in this study, the proliferation of HS Sultan cells was inhibited significantly by a combination of anti-TACI plus anti-BCMA monoclonal antibodies, and by the dual reactive BCMA-TACI monoclonal antibody, 255.7. In contrast, the myeloma cell line, RPMI 8226, was not inhibited by the anti-CD20 monoclonal antibody, whereas the TACI monoclonal antibody 248.24, and the dual reactive BCMA-TACI were quite effective. The combination of anti-TACI and anti-BCMA monoclonal antibodies appears to be effective in directly inhibiting or preventing the proliferation of lymphoma and myeloma cells. Treatment with anti-TACI plus anti-BCMA monoclonal antibodies may be especially useful for multiple myeloma where often CD20 is absent or at low levels.

### EXAMPLE 3

### Antibody Signaling Via the TACI Receptor

A Jurkat cell line was transfected with a plasmid encoding human TACI and a plasmid containing a reporter construct. The reporter construct included nucleotide sequences encoding recognition sequences for nuclear factor-kappa B and AP1 transcription factors operably linked with a gene encoding luciferase. One of the transfected Jurkat cell lines was found to be responsive to ZTNF4 in a dose dependent manner. Using this Jurkat cell line, TACI and BCMA monoclonal antibodies were tested for the ability to induce luciferase expression relative to negative control monoclonal antibodies. All monoclonal antibodies were immobilized by capture with anti-mouse IgG that had been coated onto a culture dish.

**TABLE 1**

| **Murine Monoclonal Antibody** | **Fold Induction** |
|---|---|
| IgG2a negative control | 2.0 |
| anti-BCMA-TACI (255.7) | 7.4 |
| anti-BCMA (255.4) | 1.2 |
| anti-TACI (248.24) | 7.1 |
| anti-TACI (251.10) | 2.65 |
| anti-TACI (250.13) | 2.25 |

Table 1 provides the results of these studies. Stimulation indices shown are the ratios of luciferase activity with test monoclonal antibody to the luciferase activity with no monoclonal antibody present. The highest signaling activity was displayed by BCMA-TACI monoclonal antibody 255.7, and TACI monoclonal antibody 248.24. For comparison, ZTNF4 (100 ng/ml) induced a signal with an index greater than 10. Other TACI monoclonal antibodies and a BCMA monoclonal antibody (255.4) were inactive in this assay. These results reconfirm the dual reactive nature of the BCMA-TACI monoclonal antibody 255.7, since the antibody must signal via TACI in the assay. The signaling activity displayed by TACI monoclonal antibody 248.24 correlates with its anti-tumor activity *in vivo,* a correlation that has been observed by others. See, for example, Grafton et al., Cell. Immun. 182:45 (1997), and Tutt et al., J. Imm. 161:3176 (1998).

### EXAMPLE 4

### Immunoconjugate Treatment of a Multiple Myeloma Cell Line

Various cell lines were tested with antibodies to detect the presence of TACI, BCMA, and CD20, as well as with labeled ZTNF4 ligand. As shown in Table 2, multiple myeloma cell lines expressed a relatively high level of BCMA. This was so despite a relatively low level of ZTNF4 binding. Thus, BCMA provides a suitable marker for targeting multiple myeloma cells.

**TABLE 2**

| **Human** **Cell Line** | **Cell Type** | **BCMA** **Ab** | **ZTNF4** | **TACI** **Ab** | **CD20** **Ab** |
|---|---|---|---|---|---|
| NCIH929 | Myeloma | 626 | 159 | 0 | - |
| U266 | Myeloma | 83 | 3 | 1 | - |
| RPMI8226 | Myeloma | 123 | 25 | 12 | - |
| OPM2 | Myeloma | 95 | 9 | 13 | - |
| L363 | Plasma cell leukemia | 56 | 102 | 86 | +/- |
| ARH-77 | Plasma cell leukemia | 70 | 104 | 35 | +++ |
| RL | NHL | 4 | 141 | 0 | +++ |
| MC116 | Lymphoma | 6 | 106 | 2 | +++ |
| IM9 | Lymphoma | 7 | 166 | 72 | +++ |

Anti-receptor antibodies were tested for the ability to target myeloma cells with a cytotoxic polypeptide. In this study, antibodies were diluted into standard RPMI medium in 96-well microtiter plates. Mabzap (G-α-mlgG-saporin; Advanced Targeting Systems; San Diego, CA), saporin conjugated to affinity-purified goat anti-mouse IgG, was then added at a concentration of 50 nanograms per well and incubated for 10 minutes at room temperature. Multiple myeloma cell lines were then added at a final concentration of 2,500 cells per well. Cells were incubated for 48 hours at 37°C, pulsed overnight with 1 µCi of ³thymidine per well, and harvested onto filter mats the following day.

In this study, the inhibition of proliferation was dependent upon the internalization of immunoconjugate. As shown in Table 3, anti-BCMA-TACI immunoconjugates inhibited the proliferation of multiple myeloma and plasma cell leukemia cells. A higher dose of anti-BCMA-TACI immunoconjugate was required to inhibit proliferation of NC1H929 cells, compared with the dose of anti-CD40 immunoconjugate, suggesting a relatively lower rate of internalization for anti-BCMA-TACI immunoconjugate. A low internalization rate may promote antibody-dependent cellular cytotoxicity and complement-mediated killing.

**TABLE 3**

| **Cell Line** | **Saporin** **Immunoconjugate** | **Dose for 50% Inhibition** **(µg/ml)** |
|---|---|---|
| ARH-77 | CD40 | 0.006 |
| ARH-77 | BCMA-TACI (255.7) | 1.5 |
| ARH-77 | CD40 | 0.019 |
| L363 | CD138 | 0.019 |
| L363 | BCMA-TACI (255.7) | 0.056 |
| NCIH929 | CD138 | 0.019 |
| NCIH929 | BCMA-TACI (255.7) | 170 |
| NCIH929 | BCMA (255.4) | 500 |

CLAUSE 1. Use of an antibody component that binds both the B-cell maturation antigen (BCMA) and the transmembrane activator and calcium-modulator and cyclophilin ligand-interactor (TACI) in the manufacture of a composition for inhibiting the proliferation of tumor cells.
CLAUSE 2. Use according to clause 1, wherein the composition is administered to cells cultured *in vitro.*
CLAUSE 3. Use according to clause 1, wherein the composition is a pharmaceutical composition, and wherein the pharmaceutical composition is administered to a mammalian subject, which has a tumor.
CLAUSE 4. Use according to clause 1, wherein the composition comprises an anti BCMA-TACI antibody component that is a naked BCMA-TACI antibody.
CLAUSE 5. Use according to clause 1, wherein the composition comprises an anti BCMA-TACI antibody component that is a naked BCMA-TACI antibody fragment.
CLAUSE 6. Use according to clause 1, wherein the composition comprises an immunoconjugate that comprises an anti-BCMA-TACI antibody component and a therapeutic agent.
CLAUSE 7. Use according to clause 6, wherein the therapeutic agent is selected from the group consisting of chemotherapeutic drug, cytotoxin, immunomodulator, chelator, boron compound, photoactive agent, photoactive dye, and radioisotope.
CLAUSE 8. Use according to clause 1, wherein the composition comprises an antibody fusion protein that comprises an anti-BCMA-TACI antibody component and a cytotoxic polypeptide.
CLAUSE 9. Use according to clause 1, further comprising administering a composition that comprises an antibody component, which binds to an epitope within a polypeptide consisting of amino acid residues 105 to 166 of SEQ ID NO: 4.
CLAUSE 10. Use according to clause 9, wherein the antibody component binds to a polypeptide consisting of amino acid residues 110 to 118 of SEQ ID NO: 4.
CLAUSE 11. Use of an anti-BCMA-TACI antibody component in the manufacture of a composition for inhibiting ZTNF4 activity in a mammal.
CLAUSE 12. The method of clause 11, wherein the ZTNF4 activity is associated with increased endogenous antibody production.
CLAUSE 13. Use according to clause 11, wherein the ZTNF4 activity is associated with a disorder selected from the group consisting of neoplasm, chronic lymphocytic leukemia, multiple myeloma, non-Hodgkin's lymphoma, post-transplantation lymphoproliferative disease, and light chain gammopathy.
CLAUSE 14. Use according to clause 11, wherein the ZTNF4 activity is associated with inflammation, and wherein administration of the composition decreases inflammation.
CLAUSE 15. Use of a multispecific antibody composition in the manufacture of a pharmaceutical composition for inhibiting the proliferation of tumor cells, wherein the multispecific antibody composition comprises:
   (a) an antibody component that binds the extracellular domain of the B-cell maturation antigen (BCMA), and
   (b) an antibody component that binds the extracellular domain of transmembrane activator and calcium-modulator and cyclophilin ligand interactor (TACI), wherein the anti-TACI antibody component does not bind the extracellular domain of BCMA,
   wherein the administration of the multispecific antibody composition inhibits the proliferation of tumor cells.
CLAUSE 16. Use according to clause 15, wherein the multispecific antibody composition is administered to cells cultured *in vitro.*
CLAUSE 17. Use according to clause 15, wherein the multispecific antibody composition is a pharmaceutical composition, and wherein the pharmaceutical composition is administered to a mammalian subject, which has a tumor.
CLAUSE 18. Use according to clause 15, wherein the multispecific antibody composition comprises an anti-BCMA naked antibody component and an anti-TACI naked antibody component.
CLAUSE 19. Use according to clause 15, wherein the multispecific antibody composition comprises bispecific antibodies that bind BCMA and TACI.
CLAUSE 20. Use according to clause 15, wherein at least one of the antibody components further comprises a therapeutic agent.
CLAUSE 21. Use according to clause 20, wherein the therapeutic agent is selected from the group consisting of chemotherapeutic drug, cytotoxin, immunomodulator, chelator, boron compound, photoactive agent, photoactive dye, and radioisotope.
CLAUSE 22. Use according to clause 15, wherein the multispecific antibody composition comprises: (a) an antibody fusion protein that comprises a cytotoxic polypeptide, and (b) at least one of an anti-BCMA antibody component or an anti-TACI antibody component.
CLAUSE 23. Use according to clause 15, wherein the tumor cells are lymphoma cells.
CLAUSE 24. An antibody component that specifically binds with both the B-cell maturation antigen (BCMA) and transmembrane activator and calcium-modulator and cyclophilin ligand-interactor (TACI).

## Claims

1. Use of an antibody component that binds both the B-cell maturation antigen (BCMA) and the transmembrane activator and calcium-modulator and cyclophilin ligand-interactor (TACI) for the manufacture of a composition for inhibiting the proliferation of tumor cells, wherein said antibody component binds to amino acid residues 1-48 of SEQ ID NO: 2, and to amino acid residues 30-67 or 68-154 of SEQ ID NO: 4.

2. An *in vitro* method for inhibiting the proliferation of tumour cells cultured *in vitro,* comprising administering to the cells an antibody component that binds both the B-cell maturation antigen (BCMA) and the transmembrane activator and calcium-modulator and cyclophilin ligand-interactor (TACI), wherein said antibody component binds to amino acid residues 1-48 of SEQ ID NO:2, and to amino acid residues 30-67 or 68-154 of SEQ ID NO:4.

3. Use according to Claim 1, wherein the composition comprises an anti-BCMA-TACI antibody component that is a naked BCMA-TACI antibody.

4. Use according to Claim 1, wherein the composition comprises an anti-BCMA-TACI antibody component that is a naked BCMA-TACI antibody fragment.

5. Use according to Claim 1, wherein the composition comprises an immunoconjugate that comprises an anti-BCMA-TACI antibody component and a therapeutic agent.

6. Use according to Claim 5, wherein the therapeutic agent is selected from the group consisting of chemotherapeutic drug, cytotoxin, immunomodulator, chelator, boron compound, photoactive agent, photoactive dye, and radioisotope.

7. Use according to Claim 1, wherein the composition comprises an antibody fusion protein that comprises an anti-BCMA-TACI antibody component and a cytotoxic polypeptide.

8. Use according to Claim 1, further comprising administering a composition that comprises an antibody component, which binds to an epitope within a polypeptide consisting of amino acid residues 105 to 166 of SEQ ID NO:4.

9. Use according to Claim 8, wherein the antibody component binds to a polypeptide consisting of amino acid residues 110 to 118 of SEQ ID NO:4.

10. Use of an anti-BCMA-TACI antibody component for the manufacture of a composition for treating a medical condition selected from neoplasms, chronic lymphocytic leukemia, multiple myeloma, non-Hodgkins lymphoma, carcinomas, B cell lymphomas, B cell leukemias, plasma cytomas, Epstin Barr virus-associated lymphomas, overt multiple myeloma, smoldering multiple myeloma, plasma cell leukemia, non-secretory myeloma, IgD myeloma, osteosclerotic myeloma, solitary plasmacytoma of bone, extramedullary plasmacytoma and urological neoplasm; by inhibiting ZTNF4 activity in a mammal;
wherein said antibody component binds to amino acid residues 1-48 of SEQ ID NO:2, and to amino acid residues 30-67 or 68-154 of SEQ ID NO:4.

11. Use according to Claim 10, wherein said medical condition is selected from the group consisting of neoplasm, chronic lymphocytic leukaemia, multiple myeloma, and non-Hodgkin's lymphoma.

12. An antibody component that specifically binds with both the B-cell maturation antigen (BCMA) and transmembrane activator and calcium-modulator and cyclophilin ligand-interactor (TACI), wherein said antibody component binds to amino acid residues 1-48 of SEQ ID NO:2, and to amino acid residues 30-67 or 68-154 of SEQ ID NO:4.

13. An antibody component that binds both the B-cell maturation antigen (BCMA) and the transmembrane activator and calcium-modulator and cyclophilin ligand-interactor (TACI) for use in inhibiting the proliferation of tumor cells, wherein said antibody component binds to amino acid residues 1-48 of SEQ ID NO:2, and to amino acid residues 30-67 or 68-154 of SEQ ID NO:4.

14. An anti-BCMA-TACI antibody component for use in treating a medical condition selected from neoplasms, chronic lymphocytic leukemia, multiple myeloma, non-Hodgkins lymphoma, carcinomas, B cell lymphomas, B cell leukemias, plasma cytomas, Epstin Barr virus-associated lymphomas, overt multiple myeloma, smoldering multiple myeloma, plasma cell leukemia, non-secretory myeloma, IgD myeloma, osteosclerotic myeloma, solitary plasmacytoma of bone, extramedullary plasmacytoma and urological neoplasm; by inhibiting ZTNF4 activity in a mammal;
wherein said antibody component binds to amino acid residues 1-48 of SEQ ID NO:2, and to amino acid residues 30-67 or 68-154 of SEQ ID NO:4.
